# FASCICULE DE BREVET EUROPEEN

(11) **EP 0 586 266 B1**
(45) Date de publication et mention de la délivrance du brevet: **11.06.2003**
(21) Numéro de dépôt: 93401531.4
(22) Date de dépôt: 15.06.1993
(51) Int. Cl.: C12N 15/31, C12N 1/21, C12P 21/02, C12P 21/08, C07K 14/00

(54) **Fragments d'ADN codant pour les sous-unités du récepteur de la transferrine de Neisseria meningitidis**
DNA-Fragmente, die für die Untereinheiten von dem Neisseria Meningitidis Rezeptor kodieren
DNA fragments coding for the Neisseria meningitidis receptor fragments

(30) Priorité: 19.06.1992 FR 9207493
(43) Date de publication de la demande: 09.03.1994
(73) Titulaire: Aventis Pasteur, 69367 Lyon Cedex 07 (FR); TRANSGENE S.A., 67000 Strasbourg (FR)
(72) Inventeur: Jacobs, Eric, F-67120 Dorlisheim (FR); Legrain, Michèle, F-67120 Dorlisheim (FR); Mazarin, Véronique, F-69002 Lyon (FR); Bouchon-Theisen, Bernadette, F-67100 Strasbourg (FR); Schryvers, Anthony B., Calgary, Alberta T3A 3V8 (CA); Bloch Marie-Aline, F-69005 Lyon (FR)
(74) Mandataire: Bernasconi, Jean

(56) Documents cités:
- WO-A-90/12591
- WO-A-92/03467
- INFECTION AND IMMUNITY vol. 60, no. 6, Juin 1992, pages 2391 - 2396 Stevenson P; Williams P; Griffiths E; 'Common antigenic domains in transferrin -binding protein 2 of Neisseria -Meningitidis Neisseria -Gonorhoeane and Haemophilus-Influenzae Type B.'

## Description

La présente invention a pour objet des fragments d'ADN de *Neisseria meningitidis* codant pour les sous-unités du récepteur de la transferrine ainsi qu'un procédé de fabrication de chacune des sous-unités par voie recombinante.

D'une manière générale, les méningites sont soit d'origine virale, soit d'origine bactérienne. Les bactéries principalement responsables sont : *N. meningitidis* et *Haemophilus influenzae,* respectivement impliquées dans environ 40 et 50 % des cas de méningites bactériennes.

On dénombre en France, environ 600 à 800 cas par an de méningites à *N. meningitidis*. Aux Etats-Unis, le nombre de cas s'élève à environ 2 500 à 3 000 par an.

L'espèce *N. meningitidis* est subdivisée en sérogroupes selon la nature des polysaccharides capsulaires. Bien qu'il existe une douzaine de sérogroupes, 90 % des cas de méningites sont attribuables à 3 sérogroupes : A, B et C.

Il existe des vaccins efficaces à base de polysaccharides capsulaires pour prévenir les méningites à *N. meningitidis* sérogroupes A et C. Ces polysaccharides tels quels ne sont que peu ou pas immunogéniques chez les enfants de moins de 2 ans et n'induisent pas de mémoire immunitaire. Toutefois, ces inconvénients peuvent être surmontés en conjuguant ces polysaccharides à une protéine porteuse.

Par contre, le polysaccharide de *N. meningitidis* groupe B n'est pas ou peu immunogène chez l'homme, qu'il soit sous forme conjuguée ou non. Ainsi, il apparait hautement souhaitable de rechercher un vaccin à l'encontre des méningites induites par *N. meningitidis* notamment du sérogroupe B autre qu'un vaccin à base de polysaccharide.

A cette fin, différentes protéines de la membrane externe de *N. meningitidis* ont déjà été proposées. Il s'agit en particulier du récepteur membranaire de la transferrine humaine.

D'une manière générale, la grande majorité des bactéries ont besoin de fer pour leur croissance et elles ont développé des systèmes spécifiques d'acquisition de ce métal. En ce qui concerne notamment *N. meningitidis* qui est un pathogène strict de l'homme, le fer ne peut être prélevé qu'à partir de protéines humaines de transport du fer telles que la transferrine et la lactoferrine puisque la quantité de fer sous forme libre est négligeable chez l'homme (de l'ordre de 10⁻¹⁸ M), en tout cas insuffisante pour permettre la croissance bactérienne.

Ainsi, *N. meningitidis* possède un récepteur de la transferrine humaine et un récepteur de la lactoferrine humaine qui lui permettent de fixer ces protéines chélatrices du fer et de capter par la suite le fer nécessaire à sa croissance.

Le récepteur de la transferrine de la souche *N. meningitidis* B16B6 a été purifié par Schryvers et al (WO 90/12591) à partir d'un extrait membranaire. Cette protéine telle que purifiée apparait essentiellement constituée de 2 types de polypeptides : un polypeptide d'un poids moléculaire apparent élevé de 100 kD et un polypeptide d'un poids moléculaire apparent moindre d'environ 70 kD, telles que révélés après électrophorèse sur gel de de polyacrylamide en présence de SDS.

Le produit de la purification notamment mise en oeuvre par Schryvers est par définition arbitraire et pour les besoins de la présente demande de brevet, appelé récepteur de la transferrine et les polypeptides le constituant, des sous-unités. Dans la suite du texte, les sous-unités de poids moléculaire élevé et de poids moléculaire moindre sont respectivement appelées Tbp1 et Tbp2.

Toutefois, le procédé de purification décrit par Schryvers et al ne peut pas être utilisé pour la production à grande échelle du récepteur de la transferrine. La préparation industrielle de ce récepteur sous forme purifiée passe nécessairement par une étape de production à l'aide d'un système d'expression hétérologue.

A cette fin, l'invention se propose de fournir les fragments d'ADN codant pour les sous-unités du récepteur de la transferrine de *N. meningitidis*.

D'autre part, depuis les travaux pionniers de Schryvers et al, on a découvert qu'il existait en fait au moins 2 types de souches qui différent par la constitution de leurs récepteurs de la transferrine respectifs. Ceci a été mis en évidence en étudiant des extraits membranaires de plusieurs dizaines de souches de *N. meningitidis* d'origines variées. Ces extraits membranaires ont tout d'abord été soumis à une électrophorèse sur gel de polyacrylamide en présence de SDS, puis électrotransférés sur feuilles de nitrocellulose. Ces feuilles de nitrocellulose ont été incubées :
a) en présence d'un antisérum de lapin dirigé contre le récepteur de la transferrine purifié à partir de la souche *N. meningitidis* B16B6, aussi appelée IM2394 ;
b) en présence d'un antisérum de lapin dirigé contre le récepteur de la transferrine purifié à partir de la souche *N. meningitidis* IM2169 ; ou
c) en présence de la transferrine humaine conjuguée à la peroxydase.

En ce qui concerne a) et b), la reconnaissance des sous-unités du récepteur de la transferrine est révélée par addition d'un anticorps anti-immunoglobulines de lapin couplé à la peroxydase, puis par addition du substrat de cette enzyme.

Les tableaux I et II ci-dessous indiquent le profil de certaines souches représentatives tel qu'il apparait sur gel de polyacrylamide à 7,5 % après électrophorèse en présence de SDS ; les bandes sont caractérisées par leur poids moléculaires apparents exprimés en kilodaltons (kD) :

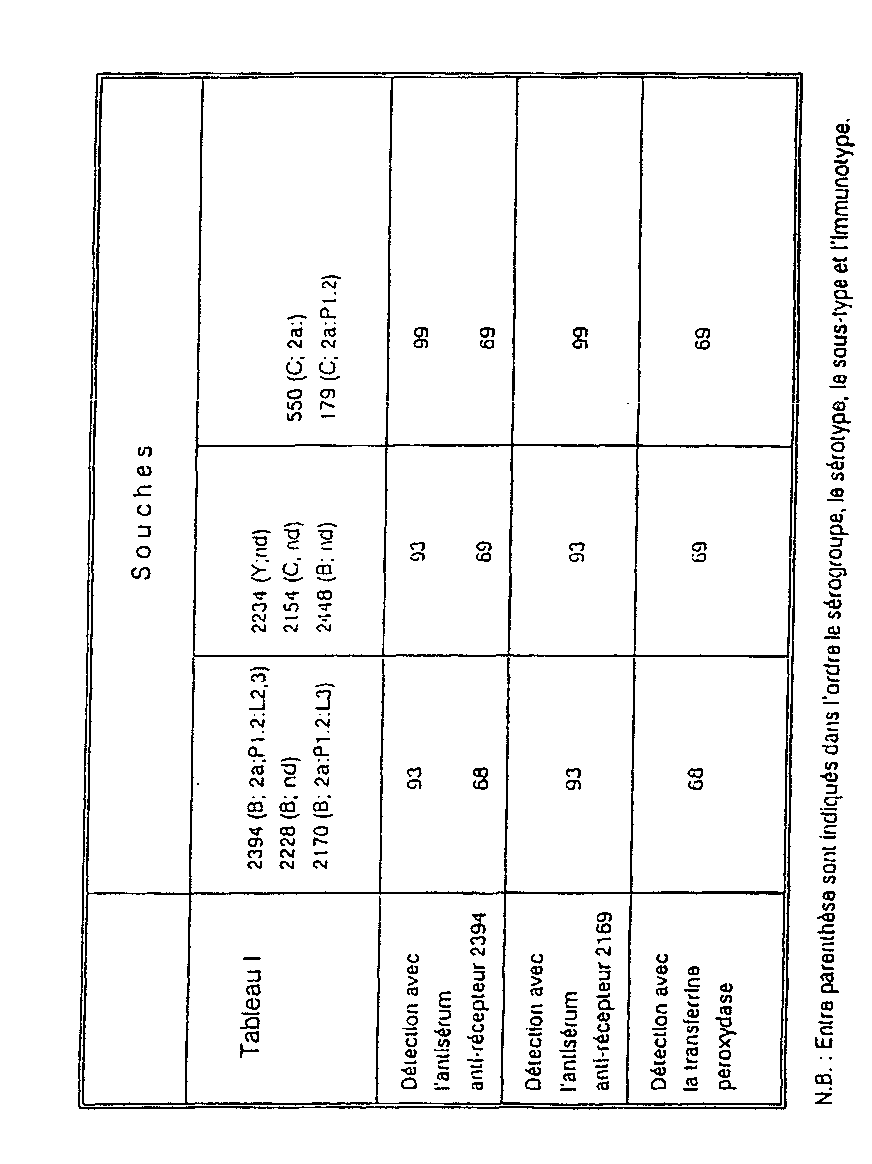

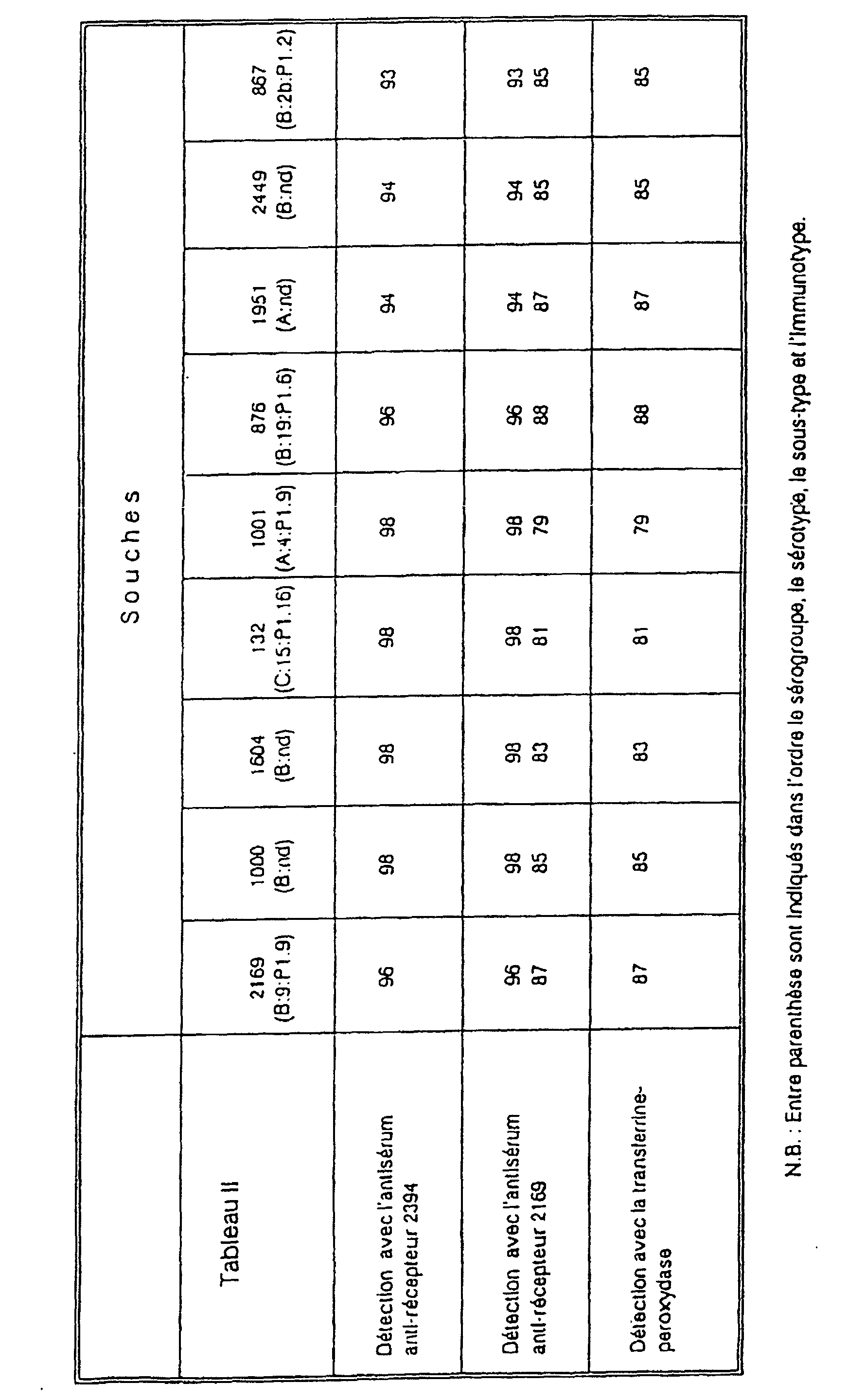

Les résultats répertoriés dans les 2 premières lignes des tableaux montrent qu'il existe 2 types de souches :

Le premier type (Tableau I) correspond à des souches qui possèdent un récepteur dont les 2 sous-unités dans les conditions expérimentales utilisées, sont reconnues par l'antisérum anti-récepteur IM2394 tandis que seule la sous-unité de haut poids moléculaire est reconnue par l'antisérum anti-récepteur IM2169.

Le second type (Tableau II) correspond à des souches qui possèdent un récepteur dont les 2 sous-unités dans les conditions expérimentales utilisées, sont reconnues par l'antisérum anti-récepteur IM2169 tandis que seule la sous-unité de haut poids moléculaire est reconnue par l'antisérum anti-récepteur IM2394.

En conséquence, il existe une diversité antigénique au niveau de la sous-unité de moindre poids moléculaire. Cette diversité est toutefois restreinte puisqu'elle se résout en 2 grands types, contrairement à ce qui est suggéré par Griffiths et al, FEMS Microbiol. Lett. (1990) 69 : 31.

En vertu de ces constatations, on pouvait supposer qu'un vaccin efficace à l'encontre de toutes les infections à *N. meningitidis* pourrait être constitué de manière suffisante, de la sous-unité de haut poids moléculaire, quelle que soit la souche d'origine du récepteur, puisque cette dernière est reconnue par les 2 types d'antisérums. Toutefois, il semble que cela ne puisse être le cas dans la mesure où la sous-unité de haut poids moléculaire ne serait pas capable d'induire la production d'anticorps de type neutralisant. Seule la plus petite des 2 sous-unités du récepteur serait capable de remplir cette fonction. Puisque cette sous-unité de moindre poids moléculaire se caractérise par une variation antigénique significative du premier type au deuxième type de souche, un seul type de récepteur de la transferrine ne devrait pas être suffisant pour vacciner contre toutes les infections à *N. meningitidis*. Par conséquent, un vaccin devra contenir au moins la sous-unité de moindre poids moléculaire de chacune des souches IM2394 et IM2169 ou de leurs équivalents respectifs et, de manière optionnelle, la sous-unité de haut poids moléculaire d'au moins une souche de *N. meningitidis*.

C'est pourquoi l'invention fournit un fragment d'ADN isolé codant pour un peptide, un polypeptide ou une protéine capable d'être reconnue par un antisérum anti-récepteur de la souche de *N. meningitidis* IM2394 ou IM2169.

Un tel fragment d'ADN peut notamment comprendre une séquence nucléotidique codant pour une séquence d'acides aminés homologue à celle telle que montrée :
- dans l'identificateur de séquence (SEQ ID NO : 1) n° 1, commençant avec le résidu cystéine en position 1 et finissant avec le résidu glutamine en position 579 ;
- dans le SEQ ID NO : 3, commençant avec le résidu acide glutamique en position 1 et finissant avec le résidu phénylalanine en position 884 ;
- dans le SEQ ID NO : 5, commençant avec le résidu acide glutamique en position 1 et finissant avec le résidu phénylalanine en position 887 ; ou
- dans le SEQ ID NO : 7, commençant avec le résidu cystéine en position 1 et finissant avec le résidu glutamine en position 691.

A titre indicatif, on précise qu'un fragment d'ADN selon l'invention peut en outre comprendre une séquence nucléotidique additionnelle codant pour n'importe quelle autre séquence d'acides aminés ; les deux séquences nucléotidiques considérées, formant un cadre ouvert de lecture de manière à coder pour une protéine hybride ou un précurseur.

De manière avantageuse, un fragment d'ADN selon l'invention peut être sélectionné parmi :
i) Un premier fragment d'ADN isolé, ayant une séquence nucléotidique codant pour une protéine ayant une séquence d'acides aminés homologue à celle telle que montrée dans le SEQ ID NO : 1, commençant avec le résidu cystéine en position 1 et finissant avec le résidu glutamine en position 579.
ii) Un deuxième fragment d'ADN isolé ayant une séquence nucléotidique codant pour une protéine ayant une séquence d'acides aminés à celle telle que montrée dans le SEQ ID NO : 3, commençant avec le résidu acide glutamique en position 1 et finissant avec le résidu phénylalanine en position 884.
iii) Un troisième fragment d'ADN isolé ayant une séquence nucléotidique codant pour une protéine ayant une séquence d'acides aminés à celle telle que montrée dans le SEQ ID NO : 5, commençant avec le résidu acide glutamique en position 1 et finissant avec le résidu phénylalanine en position 887.
iv) Un quatrième fragment d'ADN isolé ayant une séquence nucléotidique codant pour une protéine ayant une séquence d'acides aminés à celle telle que montrée dans le SEQ ID NO : 7, commençant avec le résidu cystéine en position 1 et finissant avec le résidu glutamine en position 691.

Par "séquence d'acides aminés homologue", on entend une séquence présentant un degré d'homologie d'au moins 75 %, de manière avantageuse d'au moins 80 %, de manière préférée d'au moins 90 %, de manière tout à fait préférée de 100 %, avec la séquence d'acides aminés que l'on cite en référence. On notera que le terme "homologue" tel que défini inclut le cas particulier de l'identité.

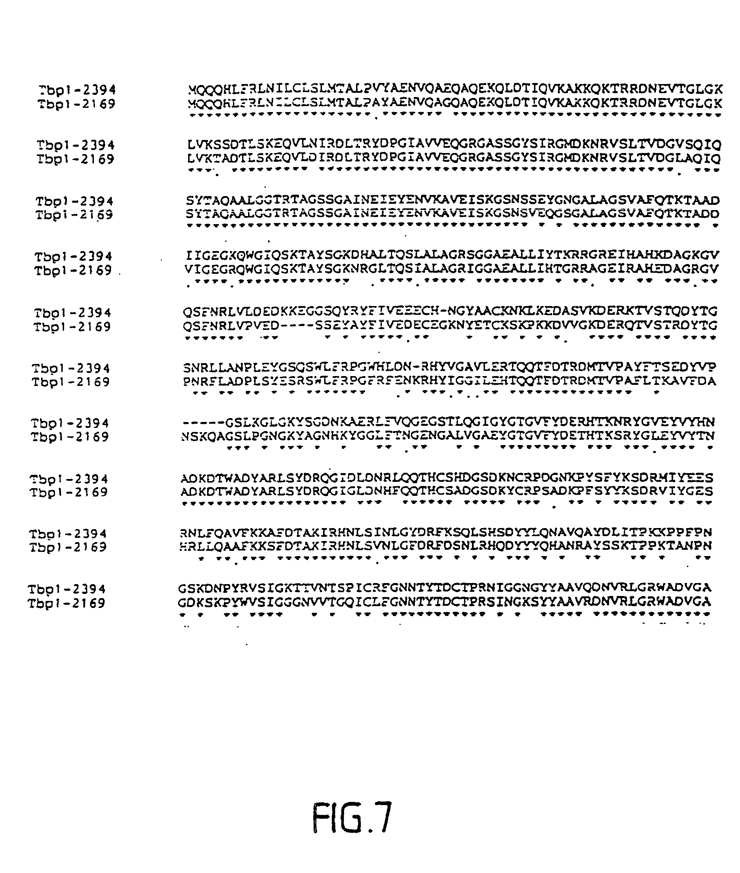

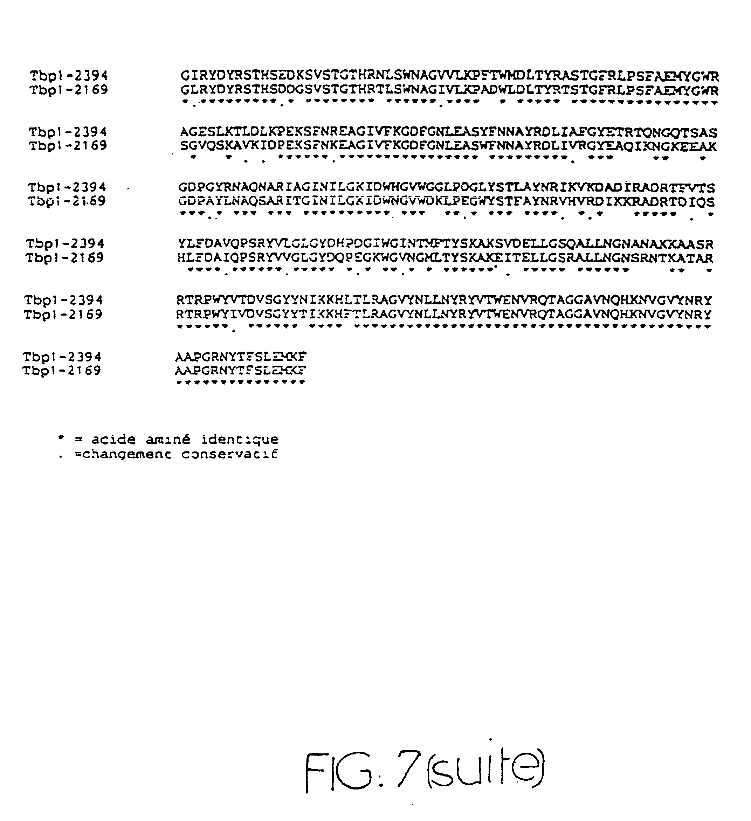

Le degré d'homologie peut être aisément calculé en alignant les séquences de manière à obtenir le degré maximal d'homologie ; pour ce faire, il peut être nécessaire d'introduire artificiellement des emplacements vacants, comme cela est illustré dans la figure 7. Une fois que l'alignement optimal est réalisé, le degré d'homologie est établi en comptabilisant toutes les positions dans lesquelles les acides aminés des deux séquences se retrouvent à l'identique, par rapport au nombre total de positions.

Il serait fastidieux de décrire des séquences homologues autrement que de manière générique, en raison du trop grand nombre de combinaisons. L'homme du métier connait toutefois les règles générales qui permettent de remplacer un acide aminé par un autre sans abolir la fonction biologique ou immunologique d'une protéine.

Un fragment d'ADN isolé et tout à fait préféré a une séquence nucléotidique codant pour :
i) La sous-unité Tbp1 de la souche IM2394 dont la séquence en acides aminés est telle que montrée dans le SEQ ID NO : 3, commençant avec le résidu acide glutamique en position 1 et finissant avec le résidu phénylalanine en position 884 ;
ii) La sous-unité Tbp2 de la souche IM2394 dont la séquence en acides aminés est montrée dans le SEQ ID NO : 1, commençant avec le résidu cystéine en position 1 et finissant avec le résidu glutamine en position 579 ;
iii) La sous-unité Tbp1 de la souche IM2169 dont la séquence en acides aminés est montrée dans le SEQ ID NO : 5, commençant avec le résidu acide glutamique en position 1 et finissant avec le résidu phénylalanine en position 887 ; ou
iv) La sous-unité Tbp2 de la souche IM2169 dont la séquence en acides aminés est montrée dans le SEQ ID NO : 7, commençant avec le résidu cystéine en position 1 et finissant avec le résidu glutamine en position 691.

Le récepteur de la transferrine étant une protéine membranaire, chacune de ses sous-unités est initialement produite sous forme d'un précurseur constitué d'un peptide signal associé en position N-terminale, à la forme mature.

C'est pourquoi l'invention a aussi pour objet un bloc d'ADN isolé codant pour un peptide signal dont la séquence d'acides aminés présente un degré d'homologie d'au moins 80 %, de manière préférée de 100 %, avec la séquence montrée dans :
i) le SEQ ID NO : 3, commençant avec le résidu méthionine en position - 24 et finissant avec le résidu alanine en position - 1 ;
ii) le SEQ ID NO : 5, commençant avec le résidu méthionine en position - 24 et finissant avec le résidu alanine en position - 1 ; ou
iii) le SEQ ID NO : 7, commençant avec le résidu méthionine en position - 20 et finissant avec le résidu alanine en position - 1.

Un fragment d'ADN selon l'invention peut être aussi sélectionné parmi un cinquième, sixième, septième et huitième fragments d'ADN codant respectivement pour un précurseur dont la séquence d'acides aminés est homologue à la séquence présentée dans le SEQ ID NO : 1, 3, 5 ou 7.

Par "fragment ou bloc d'ADN isolé" on entend un fragment ou bloc d'ADN d'origine génomique qui est i) inséré dans un vecteur viral ou plasmidique ou ii) placé sous le contrôle d'un promoteur qui lui est hétérologue.

De plus, le bloc d'ADN codant pour le peptide signal selon l'invention est, en outre, considéré comme isolé lorsque ce bloc d'ADN est associé à un fragment d'ADN codant pour une protéine hétérologue au peptide signal ; de manière à former un cadre de lecture ouvert codant pour un précurseur hybride.

L'invention concerne aussi une cassette d'expression d'un peptide, d'un polypeptide ou d'une protéine capable d'être reconnue par un antisérum anti-récepteur de la souche de *N. meningitidis* IM2394 ou IM2169, qui comprend au moins un fragment d'ADN selon l'invention, placé sous le contrôle d'éléments capables d'assurer son expression dans une cellule-hôte appropriée.

Dans la cassette d'expression, le premier, deuxième, troisième ou quatrième fragment d'ADN selon l'invention, codant pour une forme mature; peut être ou non associé à un bloc d'ADN codant pour un peptide signal, selon que l'on recherche ou non la sécrétion de la protéine. De préférence, cette sécrétion sera recherchée. Dans ce dernier cas, le bloc d'ADN peut coder pour un peptide signal homologue ou hétérologue à la forme mature, avec pour résultats respectifs, la synthèse d'un précurseur naturel ou hybride.

Les éléments indispensables à l'expression d'un fragment d'ADN selon l'invention sont un promoteur de transcription, des codons de début et de fin de traduction et, de manière optionnelle, un terminateur de transcription. Le promoteur peut être constitutif ou inductible. On indique que le fragment d'ADN codant pour la sous-unité Tbp2 de la souche IM2394 semble être toxique pour une cellule hétérologue, notamment pour *E. coli.* Dans ce cas là, il pourrait être préférable d'utiliser un promoteur inductible; par exemple, le promoteur du gène *ara*B de *Salmonella thyphimurium*.

Des éléments tels qu'un bloc d'ADN codant pour un peptide signal hétérologue (région signal) ou un promoteur existent déjà en assez grand nombre et sont connus de l'homme du métier. Ses compétences générales lui permettront de choisir une région signal ou un promoteur particulier qui seront adaptés à la cellule-hôte dans laquelle il envisage l'expression.

De manière plus particulière, on note que la sous-unité Tbp2 semble être une lipoprotéine, dans la mesure où son précurseur comporte un peptide signal caractéristique des précurseurs de lipoprotéines et du au fait qu'elle. possède une cystéine en position NH2- terminale et des d'acides aminés ayant une forte tendance à adopter une conformation de type "turn" légèrement en aval de la cystéine NH2-terminale (4 glycines). Pour référence, se reporter à Wu & Tokunaga, Current Top. Microb. Immunol. (1986) 125 : 127. La lipidation pourrait renforcer l'immunogénicité de la sous-unité Tbp2.

Ainsi, dans un système procaryote, il serait souhaitable d'obtenir la sous-unité Tbp2 soit à partir de son précurseur naturel, soit à partir d'un précurseur qui comprend un peptide signal hétérologue adapté, autorisant la lipidation ; c'est-à-dire un peptide signal d'une lipoprotéine autre que Tbp2. Un tel peptide signal a notamment pour caractéristique d'être libéré par clivage du précurseur par une signal peptidase de type II. La séquence au site de clivage du peptide signal répond à la séquence consensus (L, V, I) (A, S, T, G) (G, A) C, la cystéine (C) étant le premier acide aminé de la séquence mature. A titre d'exemple de peptide signal hétérologue, on cite notamment ceux des lipoprotéines ColE1, ColE3, Lpp, NlpA, OsmB, Pal, RlpB et TraT dont les séquences sont respectivement présentées dans les SEQ ID NO : 9 à 24, ainsi que les séquences nucléotidiques correspondantes.

En conséquence, selon un mode de réalisation particulier, une cassette d'expression selon l'invention, destinée à la production d'une protéine ayant une séquence d'acides aminés homologue à celle telle que montrée :
- dans le SEQ ID NO : 1, commençant avec le résidu cystéine en position 1 et finissant avec le résidu glucamine en position 579 ou
- dans le SEQ ID NO : 7, commençant avec le résidu cystéine en position 1 et finissant avec le résidu glutamine en position 691 ;
comprend :
i) un bloc d'ADN codant pour un peptide signal d'une lipoprotéine autre que la sous-unité Tbp2, tel que le peptide signal RlpB et
ii) un fragment d'ADN codant pour ladite protéine.

Enfin, l'invention fournit (i) un procédé de fabrication d'un peptide, d'un polypeptide ou d'une protéine capable d'être reconnu par un antisérum anti-récepteur de la souche de *N. meningitidis* IM2394 ou IM2169, selon lequel on cultive une cellule-hôte comportant une cassette d'expression selon l'invention et on recueille ledit peptide, polypeptide ou protéine à partir de la culture ; ainsi que (ii) le peptide, le polypeptide ou la protéine produit par ce procédé et (iii) les compositions pharmaceutiques, notamment vaccinales, les contenant.

Aux fins du procédé selon l'invention, la cellule-hôte peut être une cellule de mammifère, une levure ou une bactérie ; cette dernière étant préférée. Là aussi, le choix d'une lignée particulière est à la portée de l'homme du métier.

De manière alternative, une composition pharmaceutique selon l'invention peut contenir à titre de principe actif, un vecteur viral ou bactérien dans le génome duquel est inséré un fragment d'ADN selon l'invention, placé sous le contrôle des éléments nécessaires à son expression. A titre d'exemple de vecteur approprié, on cite notamment, les poxvirus, l'adénovirus et les bactéries lactiques.

Une composition pharmaceutique selon l'invention est notamment utile pour traiter ou prévenir une infection à *N. meningitidis*. Elle peut être fabriquée de manière conventionnelle. En particulier, on associe une quantité efficace d'un point de vue thérapeutique à un support ou à un diluant. Elle peut être administrée par n'importe quelle voie conventionnelle en usage dans le domaine de l'art, e.g. dans le domaine des vaccins, notamment par voie entérale ou parentérale. L'administration peut avoir lieu en dose unique ou répétée après un certain délai d'intervalle. La voie d'administration varie en fonction de divers paramètres, par exemple de l'individu traité (condition, age, etc). Une composition peut en outre contenir un adjuvant acceptable d'un point de vue pharmaceutique.

Afin de déterminer l'objet de la présente invention, on précise que les souches de *N. meningitidis* IM2394 (aussi appelée B16B6) et IM2169 (aussi appelée M982) sont publiquement disponibles auprès de la Collection de Institut Pasteur, 25 rue du Dr Roux 75015 Paris sous les numéros d'enregistrement respectifs CIP7908 et CIP7917.

Un antisérum spécifique du récepteur de la transferrine de la souche de *N. meningitidis* IM2394 ou IM2169 peut être obtenu tel que décrit dans les exemples ci-après.

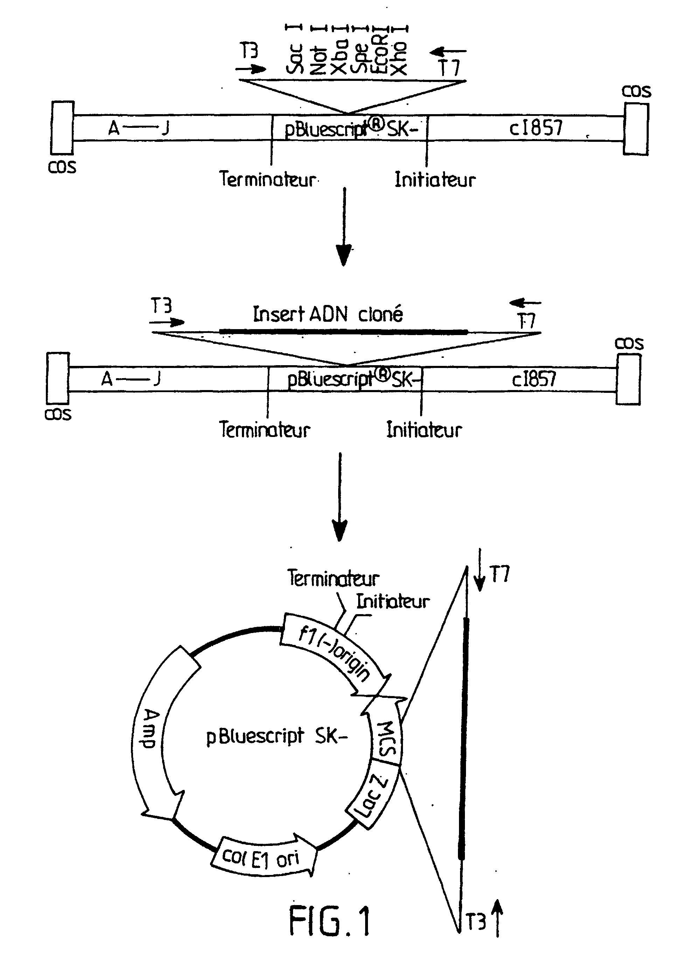

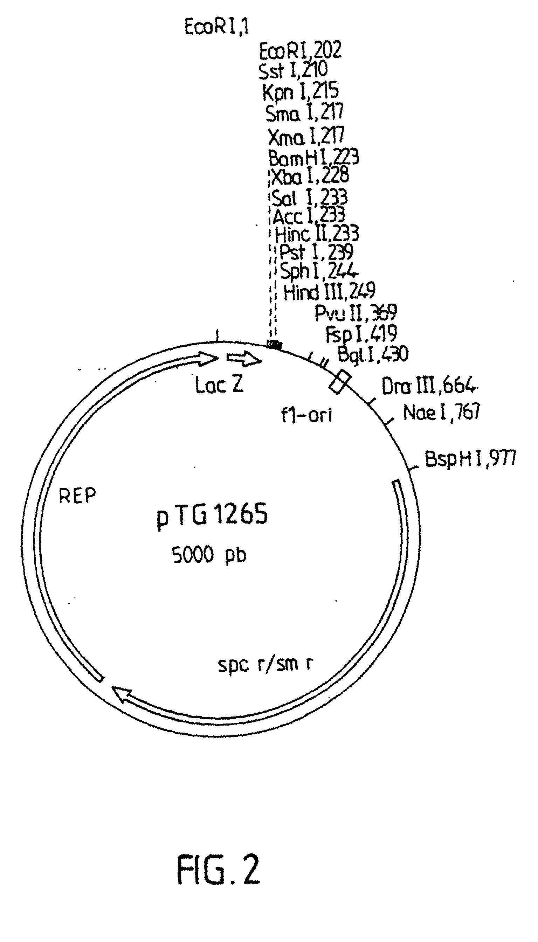

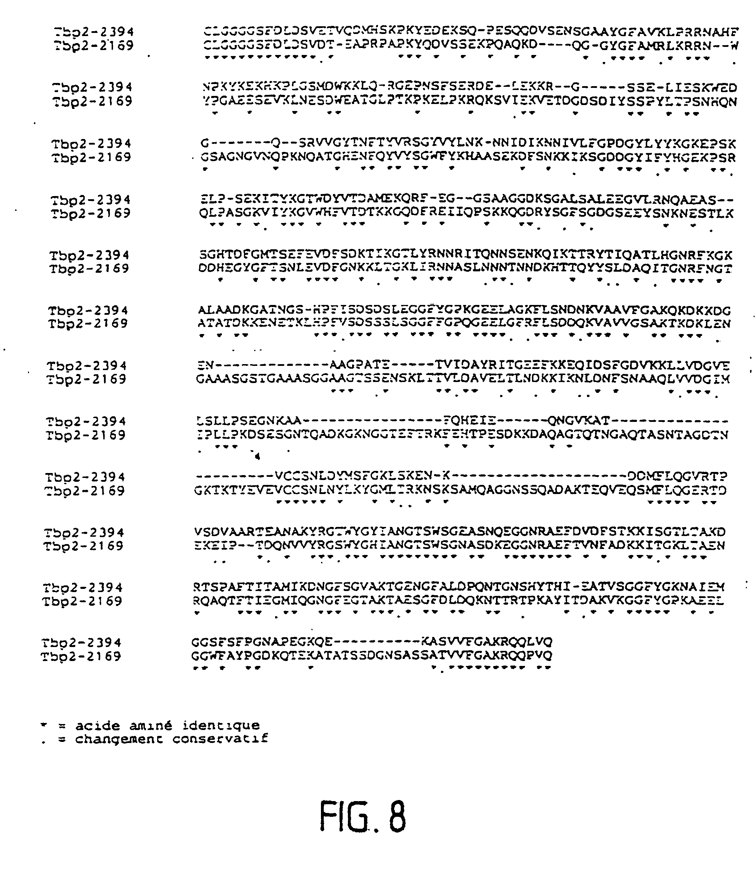

L'invention est décrite plus en détails dans les exemples ci-après et par référence aux Figures 1 à 8.

La Figure 1 représente la structure du phage lambda ZAP II et schématise la méthodologie de clonage y afférent. Lambda ZAP II est un vecteur d'insertion équipé de sites de clonage multiples localisés dans la partie plasmidique (pBluescript SK). Cette partie plasmidique peut être excisée *in vivo* par co-infection avec un phage-helper et convertie en vecteur plasmidique. Si une séquence codante est fusionnée en phase à lacZ ou si un fragment d'ADN cloné comporte un promoteur fonctionnel dans *E. coli*, il peut y avoir production d'une protéine d'intérêt qui pourra être détectée à l'aide d'anticorps spécifiques.

La Figure 2 présente la structure du plasmide pTG1265. pTG1265 dérive du plasmide pGB2 (Churchward et al, Gene (1984) 31 : 165) comme suit : pGB2 est digéré par *EcoRI* et *HindIII*, traité à la polymérase Klenow puis ligué au fragment *Sspl - PvuII* de 1 kb issu de pT7T3 184 (Mead et al, Protein Engineering (1986) 1 : 67 ; Pharmacia) qui comporte f1-ori, la séquence lacZ, les promoteurs T3 et T7 ainsi que des sites multiples de clonage.

La Figure 3 présente la carte génomique de la région d'ADN de la souche IM2394 comportant les séquences codant pour Tbp1 et Tbp2 ainsi que les différents fragments qui ont été clonés. B = *BamH1* ; E = *EcoRI* ; H *= HincII* ; R = *EcoRV* ; X = *XbaI* ; C = *ClaI*.

La Figure 4 présente la carte génomique de la région d'ADN de la souche IM2169 comportant les séquences codant pour Tbp1 et Tbp2 ainsi que les différents fragments qui ont été clonés. C = *ClaI* ; H = *HincII* ; M *= MluI* ; X = *XbaI* ; ? = position imprécise.

La Figure 5 présente la structure du plasmide para13. para13 est un plasmide capable de se répliquer dans *E. coli* qui comporte le promoteur de l'opéron arabinose BAD (ParaB) de *Salmonella typhimurium* (modifié au niveau de la TATA box), ainsi que le gène AraC. En aval du promoteur ParaB se trouve des sites multiples d'insertion. La série des plasmides para est décrite par Cagnon et al, Prot. Eng. (1991) 4 : 843.

La Figure 6 représente la méthodologie qui a été mise en oeuvre pour construire le vecteur d'expression pTG3786.

La Figure 7 compare les séquences d'acides aminés prédites des sous-unités Tbp1 des souches IM2394 et IM2169. Le degré d'homologie peut être estimé à environ 76 %.

La Figure 8 compare les séquences d'acides aminés prédites des sous-unités Tbp2 des souches IM2394 et IM2169. Le degré d'homologie peut être estimé à envinron 47 %.

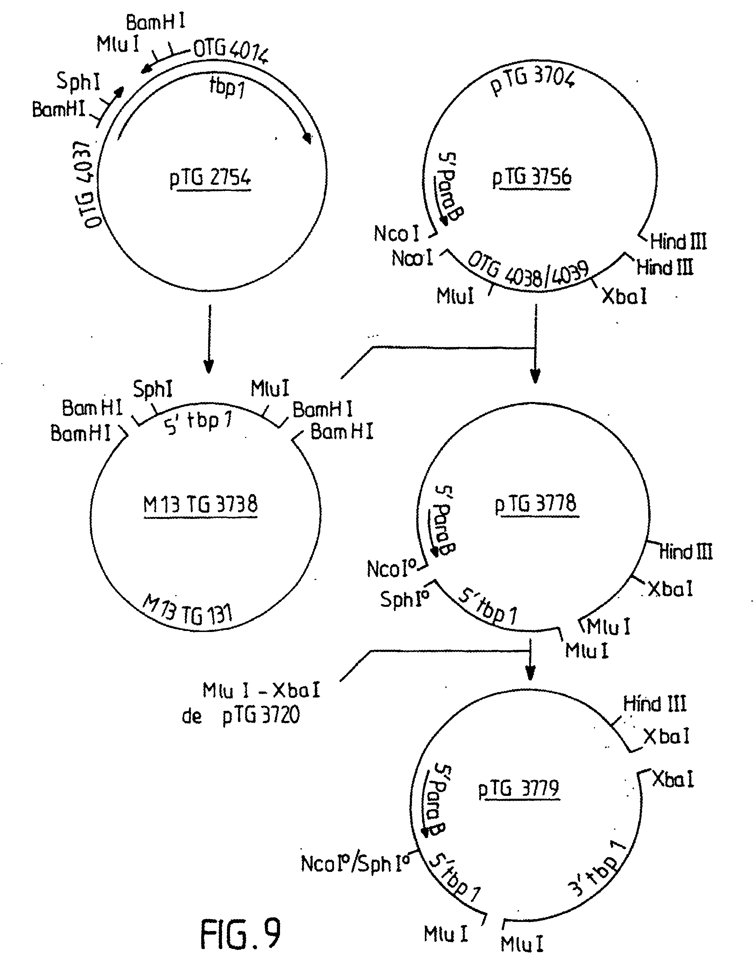

La Figure 9 représente la méthodologie qui a été mise en oeuvre pour construire le vecteur d'expression pTG3779.

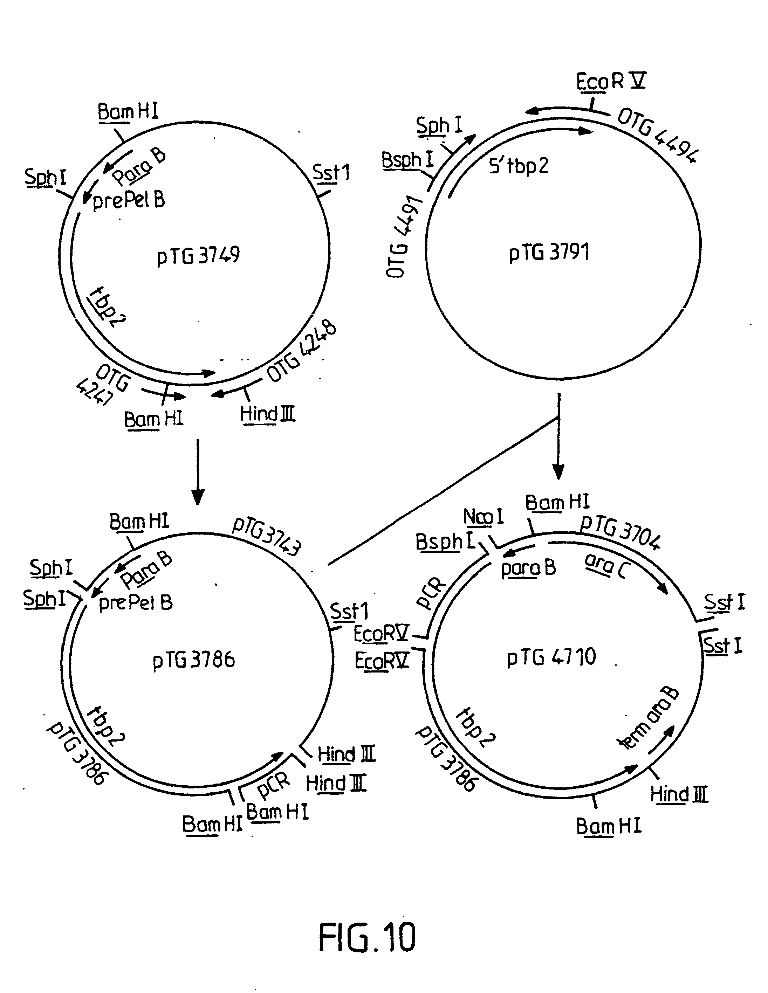

La Figure 10 représente la méthodologie qui a été mise en oeuvre pour construire le vecteur d'expression pTG4710.

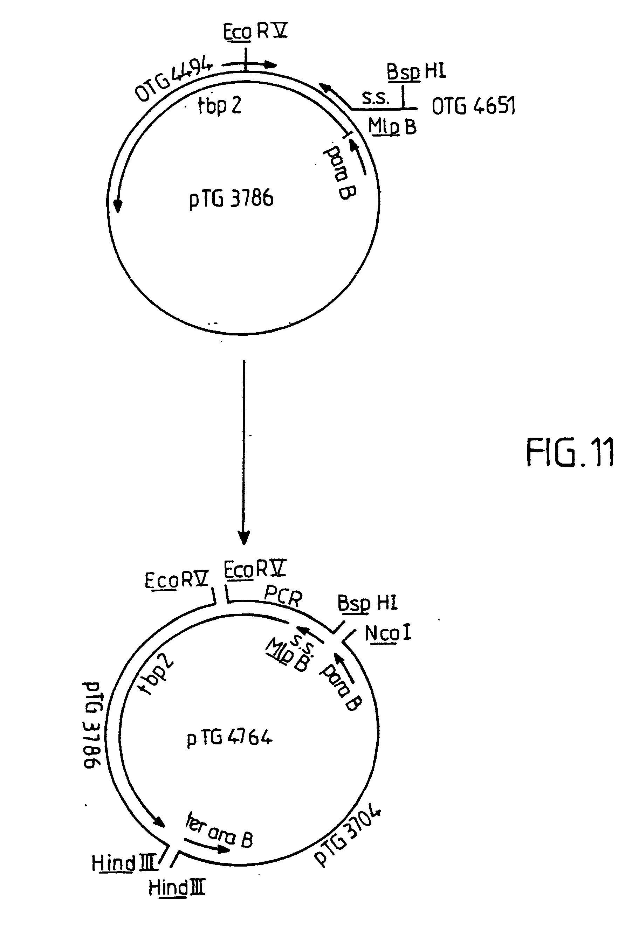

La Figure 11 représente la méthodologie qui a été mise en oeuvre pour construire le vecteur d'expression pTG4764.

### EXEMPLE 1 : Clonage des fragments d'ADN codant pour les sous-unités Tbp1 et Tbp2 du récepteur de la transferrine de la souche IM2394

### 1A - Culture de la souche et purification du récepteur de la transferrine

Un lyophilisat de la souche *N. meningitidis* IM2394 est repris dans environ 1 ml de bouillon Muller-Hinton (BMH, Difco). La suspension bactérienne est ensuite étalée sur le milieu solide Muller-Hinton contenant du sang cuit (5 %).

Après 24 hr d'incubation à 37°C dans une atmosphère contenant 10 % de CO₂, la nappe bactérienne est recueillie pour ensemencer 150 ml de BMH pH 7.2, répartis en 3 erlens de 250 ml. L'incubation est poursuivie pendant 3 hr à 37°C sous agitation. Chacune des 3 cultures ainsi réalisées permet d'ensemencer 400 ml de BMH pH 7,2 supplémenté avec 30 µM d'Ethylènediamine - Di (O-Hydroxyphenyl - acetic acid (EDDA, Sigma), qui est un agent chélatant du fer sous forme libre.

Après 16 hr de culture à 37°C sous agitation, les cultures sont contrôlées pour leur pureté par observation au microscope après une coloration de Gram. La suspension est centrifugée, le culot contenant les germes est pesé et conservé à -20°C.

La purification est mise en oeuvre essentiellement selon la méthode décrite par Schryvers et al (supra), comme suit :

Le culot bactérien est décongelé, puis remis en suspension dans 200 ml de tampon Tris HCl 50 mM, pH 8.0 (tampon A). La suspension est centrifugée pendant 20 min à 15 000 xg à 4°C. Le culot est récupéré, puis remis en suspension dans du tampon A à la concentration finale de 150 g/l. Des fractions de 150 ml sont traitées pendant 8 min à 800 bars dans un lyseur de cellules travaillant sous haute pression (Rannie, modèle 8.30H). Le lysat cellulaire ainsi obtenu est centrifugé pendant 15 min à 4°C à 15 000 xg. Le surnageant est récupéré, puis centrifugé pendant 75 min à 4°C à 200 000 xg. Après élimination du surnageant; le culot est repris dans du tampon A et après dosage de protéines selon Lowry, la concentration de la suspension est ajustée à 5 mg/ml.

A 1,4 ml de la suspension de membranes on ajoute 1,75 mg de transferrine humaine biotinylée selon le procédé décrit par Schryvers. La concentration finale de la fraction membranaire est de 4 mg/ml. Le mélange est incubé 1 heure à 37°C puis centrifugé à 100 000 xg pendant 75 minutes à 4°C. Le culot de membranes est repris par le tampon A contenant du NaCl 0,1M et incubé pendant 60 minutes à température ambiante.

Après solubilisation, on ajoute à cette suspension un certain volume de N-Lauroyl Sarkosine à 30 % (p/v) et d'EDTA 500 mM de façon que les concentrations finales en Sarkosyl et EDTA soient de 0,5 % et 5 mM respectivement. Après une incubation de 15 minutes à 37°C sous agitation, on ajoute 1 ml de résine strepavidine-agarose (Pierce) préalablement lavée en tampon A. La suspension est incubée 15 minutes à température ambiante puis centrifugée à 1 000 xg pendant 10 minutes. La résine est ensuite conditionnée dans une colonne et l'éluat direct est éliminé.

La résine est lavée par 3 volumes de colonne de tampon Tris-HCl 50 mM pH 8.0 contenant NaCl 1M, EDTA 10 mM Sarkosyl 0,5 % (tampon B) puis par un volume de colonne de tampon B contenant de la guanidine-HCl 750 mM. Le récepteur de la transferrine est ensuite élué par le tampon B contenant de la guanidine-HCl 2M. L'éluat est collecté en fractions, dans des tubes contenant un volume identique de Tris HCl 50 mM, pH 8.0, NaCl 1M. La densité optique à 280 nm de l'éluat est mesurée en sortie de colonne à l'aide d'un détecteur UV.

Les fractions correspondant au pic d'élution sont recueillies, dialysées contre du tampon phosphate 10 mM, pH 8,0 contenant du Sarkosyl 0,05 % et lyophilisées Le lyophilisât est repris dans de l'eau à une concentration 10 fois supérieure. La solution est dialysée une seconde fois contre du tampon phosphate 50 mM pH 8,0 contenant du Sarkosyl 0,05 % (tampon C) puis la solution est filtrée sur une membrane de porosité 0,22 µm.

Le contenu en protéines est déterminé et ajusté à 1 mg/ml par addition de tampon C, sous conditions aseptiques. Cette préparation est conservée à -70°C.

### 1B - Préparation d'un antisérum spécifique du récepteur de la transferrine

Des lapins néo-zélandais albinos reçoivent par voie sous-cutanée et intramusculaire 100 µg du récepteur IM2394 en présence d'adjuvant complet de Freund. 21 jours et 42 jours après la première injection, les lapins reçoivent à nouveau 100 µg du récepteur purifié mais ces fois-ci en présence d'adjuvant incomplet de Freund. 15 jours après la dernière injection, le sérum des animaux est prélevé, puis décomplémenté et filtré sur une membrane de porosité 0,45 µm. Le filtrat est par la suite épuisé par contact avec la souche IM2394 qui pour se faire, a été cultivée au préalable en présence de fer sous forme libre (dans ces conditions, la synthèse du récepteur de la transferrine est réprimée). Les modalités de contact sont comme suit : 10 ml du filtrat sont ajoutés à 10¹⁰ cfu (unités formant des colonies) d'une culture de la souche IM2394. L'adsorption est poursuivie une nuit à 4°C, sous agitation. Les bactéries sont ensuite éliminées par centrifugation. Le surnageant est récupéré puis soumis à nouveau à 2 opérations d'adsorption successives comme précédemment décrit.

### 1C - Détermination des séquences peptidiques permettant l'identification des fragments d'ADN.

Des fractions aliquotes du matériel obtenu en 1A sont séchées puis resolubilisées dans le tampon de Laemmli deux fois concentré (Tris 65mM, SDS 3 %, glycérol 10 %, 2-mercaptoéthanol 5 %). On ajoute un volume d'eau équivalent.

Après sonication, le matériel est chauffé à 90°C pendant 2 minutes, puis soumis à une électrophorèse sur gel de polyacrylamide. Les sous-unités ainsi séparées sont transférées sur membrane PVDF (Immobilon, Millipore) pendant 16 heures à 400 mA en tampon Tris borate 50 mM, pH 8,3. Les sous-unités électrotransférées sont colorées à l'amido black et les bandes correspondant à Tbp1 et Tbp2 sont récupérées et soumises au microséquençage de l'extrémité N-terminale.

Ceci est répété plusieurs fois pour établir les séquences consensus N-terminales suivantes :

Afin de séquencer des régions internes de Tbp2, la protéine sur membrane PVDF est soumise à digestion par la trypsine en tampon Tris 0,1 M pH 8,2. Après 4 heures de réaction à 37°C, les peptides sont extraits par de l'acide formique 70 % puis par de l'acide trifluoroacétique (TFA) 0,1 %. Ces peptides sont ensuite séparés par HPLC.

Pour Tbp2 IM2394, les séquences internes qui ont été établies sont les suivantes :

### 1D - Préparation de l'ADN génomique.

Le culot bactérien obtenu en 1A est resuspendu dans environ 25 ml de solution A (Tris HCI 25 mM, pH 8 contenant 50 mM de glucose et 10 mM d'EDTA) additionnée de 10 mg de protéinase K. Le mélange est laissé 10 minutes à température ambiante.

Puis on ajoute 12,5 ml de solution A contenant 10 mg de lysosyme. Une nouvelle fois, le mélange est laissé 10 minutes à température ambiante. On complète alors par 0,5 ml de sarkosyl 10 %. Le mélange est incubé 10 minutes à + 4°C.

On ajoute ensuite 2 mg de RNAse et on laisse l'incubation se poursuivre 90 minutes à 37°C. L'ADN est purifié par quatre extractions phénoliques successives. L'ADN présent dans la dernière phase aqueuse est précipité par l'éthanol. L'ADN de haut poids moléculaire est obtenu par séparation sur gradient de CsCl.

### 1E - Clonage.

Une première banque d'ADN a été réalisée dans le vecteur lambda ZAP (Figure 1), comme suit :

Une préparation d'ADN génomique a été fragmentée aux ultra-sons. Les extrémités des fragments ainsi obtenus ont été rendues franches par traitement à la T₄ polymérase. Les fragments ont été méthylés. Après méthylation, les fragments ont été liés à des adaptateurs *EcoRI*, traités par *EcoRI* puis insérés dans le site *EcoRI* du phage lambda ZAP II (Stratagène).

La souche *E. coli* XL1-Blue (Stratagène) a été infectée avec la banque d'ADN ainsi préparée. Les plages de lyse blanches (présence de phages recombinants) ont été testées à l'aide d'un antisérum spécifique du récepteur de la transferrine de la souche IM2394 préparé tel que décrit en 1B. Ceci a permis d'identifier deux clones lambda ZAP II. Les plasmides pBluescript contenus dans ces clones ont été excisés par co-infection avec le phage-"helper" et ont été appelés pBMT1 et pBMT2.

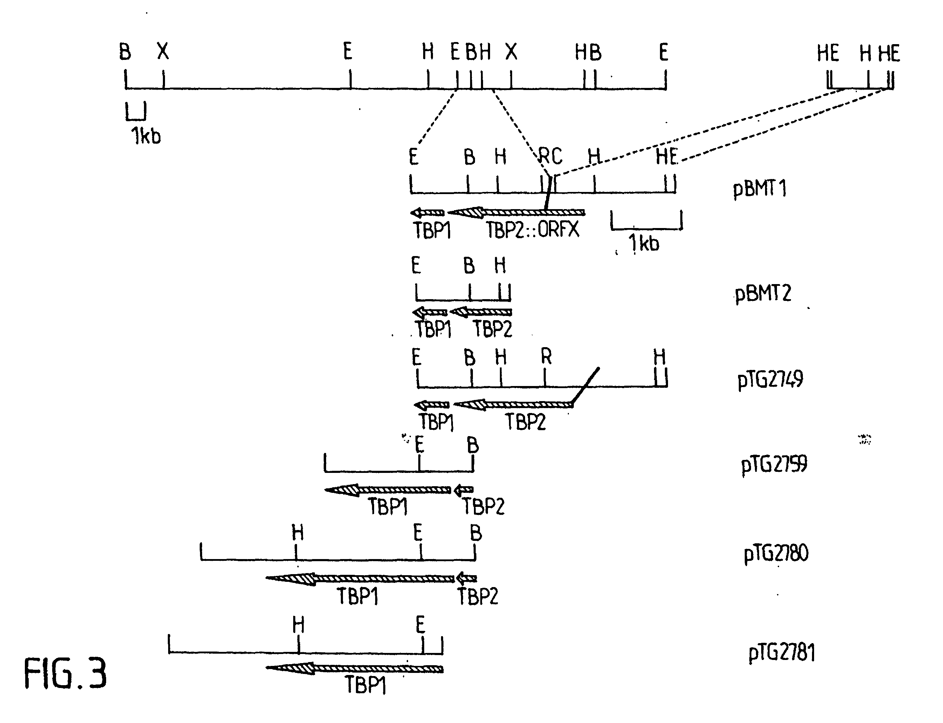

Les plasmides pBMT1 et pBMT2 contiennent chacun un fragment *EcoRI - EcoRI* respectivement de 3,8 kb et 1,3 kb. Ils sont présentés dans la Figure 3.

Le séquençage de l'insert *EcoRI - EcoRI* de pBMT1 a été mis en oeuvre selon la méthode de shotgun (Bankier et Barrell, Biochemistry (1983) B5 : 508), comme suit :

L'insert *EcoRI - EcoRI* de pBMT1 a été purifié puis fragmenté aux ultra-sons. Les extrémités des fragments ainsi obtenus ont été rendues franches par traitement à la T₄ polymérase. Les fragments ainsi traités ont été introduits dans un site du phage M13TG131 (décrit dans Kieny et al, Gene (1983) 26 : 91). Environ 200 clones issus de cette préparation ont été séquencés. L'analyse de ces séquences par ordinateur a permis de reconstituer la séquence complète de l'insert *EcoRI - EcoRI* de pBMT1.

La séquence codant pour l'extrémité N-terminale de Tbp1 a été localisée comme le montre la Figure 3. Compte tenu de la masse moléculaire de Tbp1, il était clair que cet insert ne comportait pas le fragment d'ADN complet codant pour Tbp1. En amont de l'extrémité 5' du gène *tbp1*, on a mis en évidence un cadre de lecture ouvert mais il n'a pas été possible d'identifier clairement une région codant pour l'extrémité N-terminale du gène *tbp2*.

Le microséquençage de régions internes de Tbp2 a donc été entrepris comme reporté précédemment en 1C. Les séquences internes qui étaient localisées vers l'extrémité C-terminale, correspondaient bien à la partie 3' du cadre de lecture ouvert en amont de *tbpl.*

D'autre part, l'ADN génomique de la souche IM2394, préalablement digéré par *HincII* a été analysé par Southern blot à l'aide d'une sonde d'ADN radioactive correspondant à la zone *HincII - HincII* de 1,5 kb de l'insert de 3,8 kb de pBMT1 ; deux bandes ont été ainsi révélées. Ceci a permis de démontrer que l'insert porté par pBMT1 résultait d'un assemblage artéfactuel de séquences issues de deux loci distincts. La séquence 5' de *tbp2* était donc absente.

La banque d'ADN génomique en lambda ZAP précédemment décrite a été criblée de nouveau ; cette fois-ci en utilisant l'insert *EcoRI - EcoRI* de pBMT2 comme sonde. 29 candidats ont été retenus parmi environ 200 000 plages testées. Seul le plasmide dérivé pTG2749 semblait posséder un insert nouveau par rapport à pBMT1 et pBMT2. L'insert de pTG2749 est tel que représenté dans la Figure 3. La région de l'insert en amont du site *EcoRV* (région *EcoRV - EcoRI*) a été sous-clonée dans M13TG131 et séquencée par la méthode de Sanger et al, PNAS (1977) 74 : 5463 à l'aide de primers synthétiques. La séquence correspondant à l'extrémité N-terminale de Tbp2 a été ainsi retrouvée.

La séquence du fragment d'ADN codant pour Tbp2 de la souche IM2394 est présentée dans le SEQ ID NO : 1 ainsi que la séquence d'acides aminés correspondante (SEQ ID NO: 2).

Juste en amont de la séquence codant pour Tbp2 mature, l'insert de pTG2749 comporte une région génomique distincte issue d'un autre locus. La aussi, il s'agit d'une artéfact de clonage analogue à celui mis en évidence dans le cas de pBMT1.

Compte tenu des réarrangements observés et de l'absence de séquences 3' de *tbp1* et 5' de *tbp2*, la banque d'ADN génomique construite en lambda ZAP a été jugée inadaptée pour la poursuite du clonage.

Une deuxième banque d'ADN génomique a donc été construite dans un plasmide à faible nombre de copies, comme suit : une préparation d'ADN génomique a été partiellement digérée par *Sau3A*. Des fragments d'ADN d'environ 4 à 6 kb ont été purifiés après fractionnement en gradient de sucrose et insérés dans le site *BamHI* du plasmide pTG1265. Cette préparation plasmidique a servi à transformer la souche d'*E. coli* 5K. On a estimé que cette banque contenait environ 18 000 clones indépendants.

Environ 50 000 clones de la deuxième banque ont été testés à l'aide d'une sonde radioactive correspondant à l'insert *EcoRI - EcoRI* de pBMT2. Un seul clone a été révélé ; soit le plasmide pTG2759 qui possède un insert de 1,8 kb. La taille de cette insert a été jugée insuffisante pour contenir le gène complet codant pour Tbp1.

Une troisième banque d'ADN a été construite selon la méthode décrite au paragraphe précédent à l'exception de la souche d'*E. coli* 5K qui a été remplacée par la souche d'*E. coli* SURE (Stratagène). On a estimé que cette banque contenait environ 60 000 clones indépendants.

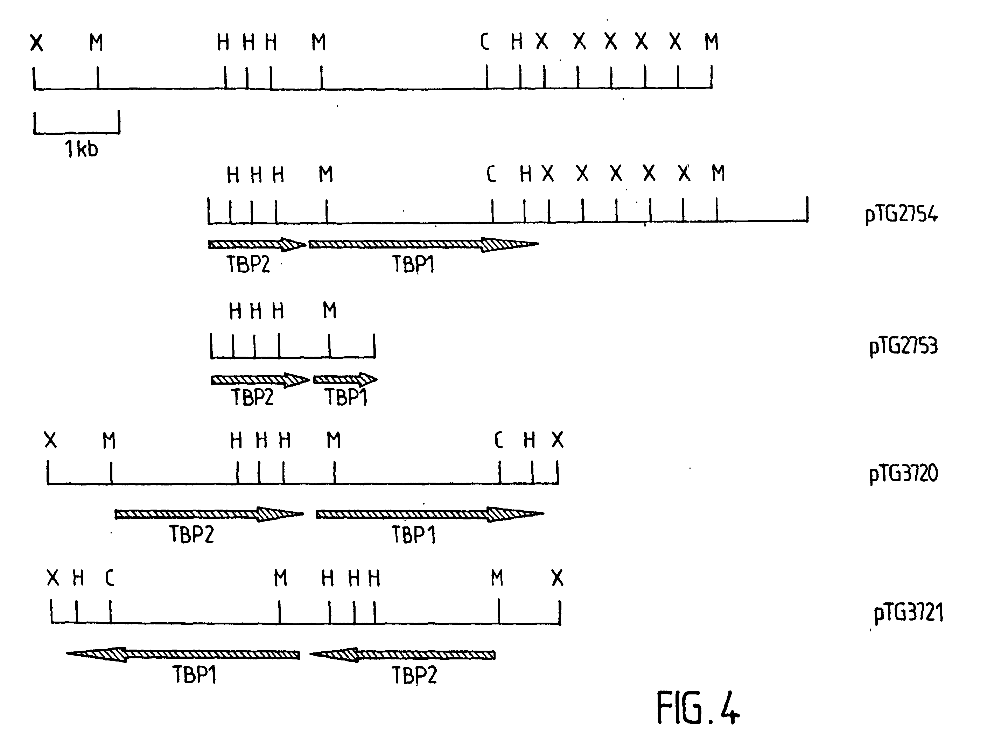

Environ 70 000 clones de la troisième banque d'ADN ont été testés à l'aide d'une sonde radioactive correspondant au fragment *MluI - HincII* de 2,4 kb issu de l'insert de pTG2754 décrit dans l'Exemple 2 ci-après et représenté dans la Figure 4. Deux clones ont été révélés, soient les plasmides pTG2780 et pTG2781, représentés dans la Figure 3.

La séquence des inserts de pTG2780 et pTG2781 a été établie selon la méthode de Sanger. Elle est présentée dans le SEQ ID NO : 3 ainsi que la séquence d'acides aminés correspondante. (SEQ ID NO:4).

Une quatrième banque a été construite. Le DNA génomique a été digéré par Sau3A et une fraction contenant des fragments d'environ 7 kb a été purifiée sur gradient de sucrose. Cette fraction contenait un fragment correspondant au locus *tbp1,2* car elle était reconnue par une sonde d'ADN spécifique de *tbp2*. Après digestion par *EcoRV* et *Xbal* et ligation à pTG1265 digéré par *Smal* et *Xbal, E coli* 5K a été transformée. Un criblage des clones à l'aide d'une sonde spécifique de *tbp2* a été réalisé. Parmi une série de clones positifs, le plasmide pTG3791 a été étudié en particulier et s'est avéré contenir des séquences 5' *tbp2* incluant la séquence codant pour le peptide signal putatif de Tbp2.

### EXEMPLE 2 : Clonage des fragments d'ADN codant pour les sous-unités Tbp1 et Tbp2 du récepteur de la transferrine de la souche IN2169.

**2A** - La culture de la souche IM2169 et la purification du récepteur de la transferrine ont été effectuées dans des conditions identiques à celles décrites dans l'Exemple 1A.
**2B -** La préparation d'un antisérum anti-récepteur de la souche IM2169 a été réalisée selon le protocole décrit dans l'Exemple 1B.
**2C** - Les séquences peptidiques permettant l'identification des fragments d'ADN ont été déterminées selon la méthode reportée dans l'Exemple 1C. Les microséquences qui ont été établies sont les suivantes.

### Séquence consensus de l'extrémité N-terminale de Tbp1 :

### Séquences des peptides internes de Tbp1 :

### Séquence consensus de l'extrémité N-terminale de Tbp2 :

### Séquences des peptides internes de Tbp2 :

### 2D - La préparation de l'ADN génomique de la souche IM2169 a été réalisée selon le protocole décrit dans l'Exemple 1D.

### 2E - Clonage

Une première banque d'ADN génomique (fragments d'ADN *Sau3A* partiel ; pTG1265 *E. coli* 5K) a été construite comme précédemment décrit dans l'Exemple 1. On a estimé que cette banque contenait environ 40 000 clones indépendants, dont environ 70 % possédaient un insert de 4-6 kb.

130 000 clones de cette banque ont été testés à l'aide d'une sonde radioactive correspondant à l'insert *EcoRI* - *EcoRI* de pBMT2. 42 clones ont été analysés, parmi lesquels 2 ont été retenus : les plasmides pTG2753 et pTG2754 qui sont tels que montrés dans la Figure 4. Les analyses en Southern blot ont montré que les cartes de restriction des inserts de pTG2753 et pTG2754 correspondaient à la carte de restriction de l'ADN génomique.

La détermination des séquences nucléotidiques et la recherche des régions codant pour les extrémités N-terminales et les régions internes ont démontré que :
- l'insert de 1,9 kb de pTG2753 contient la partie 3' du gène *tbp2* et la partie 5' du gène *tbp1* ; et
- l'insert de pTG2754 contient la partie 3' du gène *tbp2* et les parties 5' et 3' du gène *tbp1*, en rupture de phase.

Cette première banque n'a donc pas permis de cloner des fragments d'ADN complets codant pour Tbp1 ou Tbp2.

Une deuxième banque génomique a été construite comme précédemment mais à partir d'ADN génomique digéré par *XbaI*. Les fragments d'ADN ont été purifiés après fractionnement en gradient de sucrose. Chaque fraction (d'environ 500 µl) a été testée par Southern blot avec une sonde radioactive correspondant à l'extrémité 3' de *tbp1* (fragment de l'insert de pTG2754). La fraction présentant une réaction d'hybridation et contenant des fragments d'environ 6 kb a été clonée dans pTG1265. La souche *E. coli* 5K a été transformée.

Environ 2 400 clones de cette banque ont été testés à l'aide d'une sonde radioactive correspondant au fragment *HincII - MluI* de 0,6 kb issu de pTG2754. Cinq clones ont été caractérisés, parmi lesquels 2 ont été retenus : soient pTG3720 et pTG3721, tels que montrés dans la Figure 4, qui contiennent tous deux les gènes *tbp1* et *tbp2*.

Afin de compléter la séquence nucléotidique codant pour Tbp1, l'insert de pTG3720 a été séquencé dans la région où se situait la rupture de phase découverte dans l'insert de pTG2754. Ce séquençage a permis de mettre en évidence que la rupture de phase de l'insert de pTG2754 était due à une délétion de 22 bp. La séquence complète du fragment d'ADN est telle que montrée dans le SEQ ID NO 5.

Le séquençage de l'insert de pTG3720 a été poursuivi pour établir la séquence de *tbp2*. Celle-ci a bien été identifiée ; mais là aussi une rupture de phase a été constatée.

Finalement la séquence de *tbp2* a été déterminée à partir du plasmide pTG3721. Elle est telle que montrée dans le SEQ ID NO : 7.

### EXEMPLE 3 : Expression du fragment d'ADN codant pour la sous-unité Tbp2 de la souche IM2394.

### 3A. Construction du vecteur d'expression pTG3749.

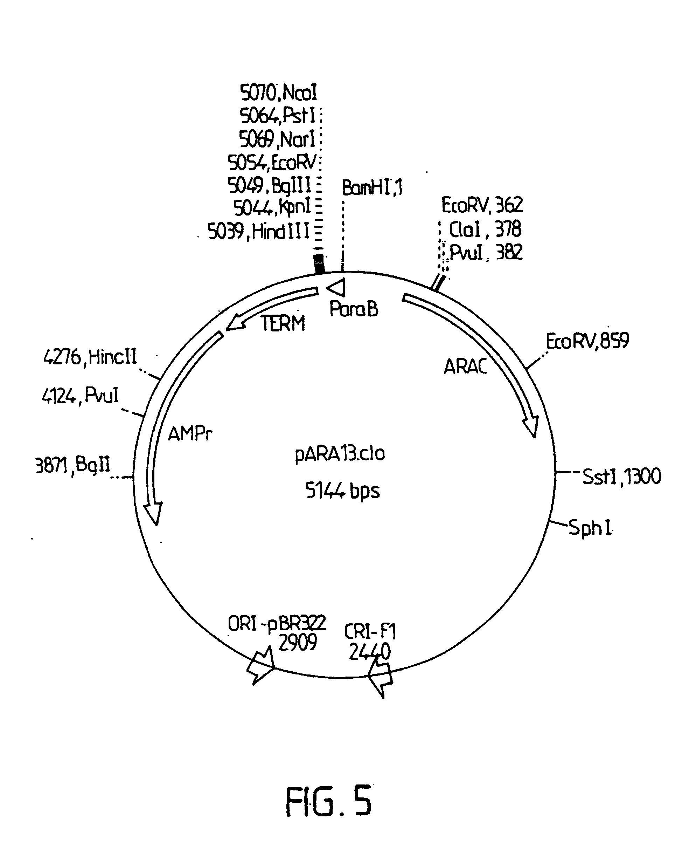

Le site *SphI* du plasmide para13 (Figure 5 ; Cagnon et al, Prot. Eng. (1991) 4 : 843) a été détruit par traitement à la polymérase Klenow, pour donner le plasmide pTG3704. pTG3704 a été linéarisé par coupure *NcoI,* traité à la polymérase Klenow pour rendre les extrémités franches, puis digéré par *HindIII*.

D'autre part, on a synthétisé les oligonucléotides OTG4015 et OTG4016 que l'on a appariés.

Le fragment d'ADN double brin OTG4015/OTG4016 a été inséré dans para13 traité comme précédemment décrit, pour donner le plasmide pTG3717 dans lequel on avait reconstitué la séquence codant pour la partie N-terminale du précurseur de la protéine PelB d'*Erwinia carotovora* (Lei et al, J. Bact. (1987) 169 : 4379) ; Soit : (en souligné, aparaissent les extrémités de pTG3704)

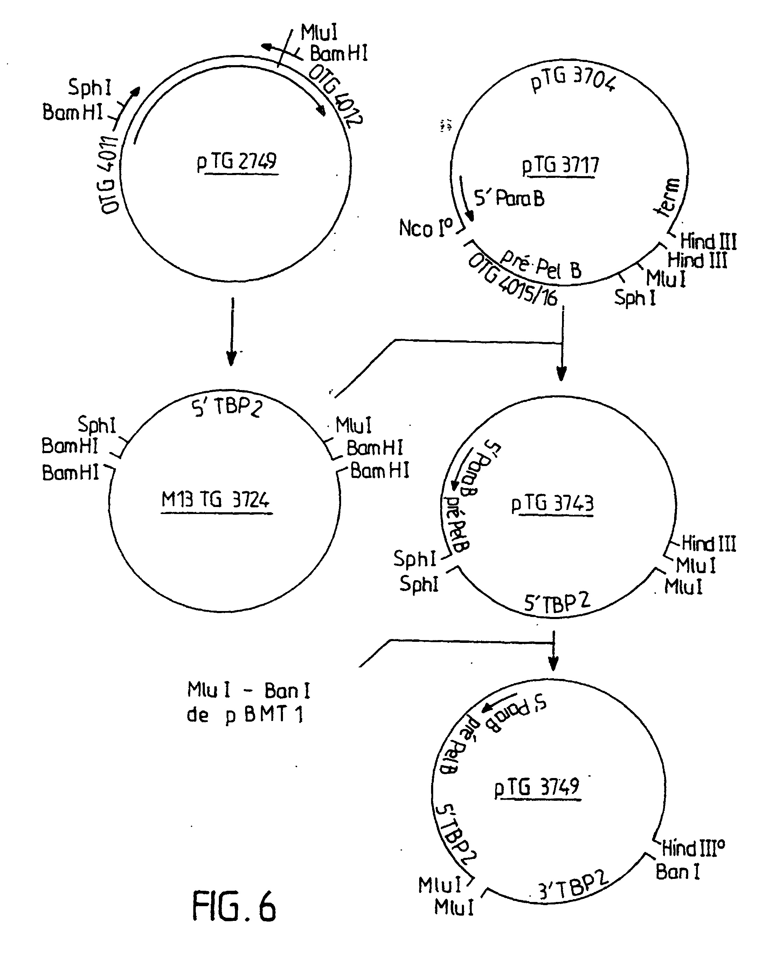

A partir du plasmide pTG2749, on a généré par PCR à l'aide des amorces OTG4011 et OTG 4012, un fragment incluant la région codant pour la partie N-terminale de Tbp2, jusqu'au site *MluI* interne, tel que montré dans la Figure 6.

Le fragment généré par PCR a été digéré par *BamHI*, puis inséré dans le site *BamHI* du phage M13TG131, pour donner M13TG3724. La séquence de ce fragment a été vérifiée par séquençage.

A partir de M13TG3724, on a récupéré la région codant pour la partie N-terminale de Tbp2 sous forme d'un fragment *SphI - MluI* que l'on a ensuite inséré dans pTG3717 préalablement digéré par *SphI* et *MluI*, pour donner le plasmide pTG3743.

A partir du plasmide pBMT1, on a récupéré la région codant pour la partie C-terminale de Tbp2 sous forme d'un fragment *MluI - BanI* dont l'extrémité cohésive *BanI* avait été rendue franche par traitement à la polymérase Klenow. On a inséré ce fragment dans pTG3743 préalablement digéré par *HindIII*, traité à la polymérase Klenow et finalement digéré par *MluI*. On a ainsi obtenu le plasmide pTG3749.

### 3B. Production de la sous-unité Tbp2.

*E. coli* MC1061 (Casadaban & Cohen, J. Mol. Biol. (1980) 138 : 179) est transformée par pTG3749 puis mise en culture à 37°C, en milieu LB supplémenté avec 2 g/l de glycérol et 100 µg/ml d'ampicilline. A la culture en phase exponentielle, on ajoute 0,2 g/l d'arabinose. L'incubation a été poursuivie durant 6 hr supplémentaires. L'expression a été observée moins d'une heure après l'addition d'arabinose.

L'électrophorèse sur gel d'acrylamide d'un échantillon du lysat cellulaire total a mis en évidence la présence d'une protéine d'environ 70 kD qui est capable de fixer la transferrine humaine marquée à la peroxydase.

### EXEMPLE 4 : Expression du fragment d'ADN codant pour la sous-unité Tbp2 de la souche IM2169.

### 4A. Construction du vecteur d'expression pTG3779.

Un fragment synthétique constitué des oligonucléotides OTG4038 et OTG4039 préalablement appariés, a été inséré dans le plasmide pTG3704 digéré par *NcoI* et *HindIII*, générant ainsi le plasmide pTG3756.

A partir du plasmide pTG2754, on a généré par PCR à l'aide des amorces OTG4037 et OTG4014 un fragment incluant la région codant pour l'extrémité N-terminale du précurseur de Tbp1 jusqu'au site *MluI.*

Ce fragment PCR a été digéré par *BamHI* et cloné dans le site *BamHI* de M13TG131 pour générer M13TG3738. La séquence de ce fragment a été vérifiée.

M13TG3738 a ensuite été linéarisé par *SphI*, traité à la T4 DNA polymérase pour rendre les extrémités franches, puis digéré par *Mlul* afin d'isoler le fragment porteur de la région codant pour l'extrémité N-terminale du précurseur de Tbp1.

Ce fragment a été inséré dans pTG3756 digéré par *NcoI,* traité à la T4 DNA polymérase puis digéré par *MluI*, pour générer le plasmide pTG3778. La séquence de la jonction *NcoI°*/*SphI°* a été vérifiée.

Le fragment *MluI - XbaI* de pTG3720 codant pour la majeure partie de Tbp1 (*3'tbp1*) a été inséré dans le plasmide pTG3778. Le plasmide final ainsi obtenu est le plasmide pTG3779.

### 4B. Production de la sous-unité Tbp1.

*E. coli* MC1061 a été transformé par pTG3779 puis mise en culture à 37°C en milieu LB. A la culture en phase exponentielle, on a ajouté 0,2 g/l d'arabinose. L'incubation a été poursuivie durant 4 heures.

L'électrophorèse sur gel d'acrylamide d'un échantillon du lysat cellulaire total a mis en évidence la présence d'une protéine d'environ 100 kD qui est reconnue par les anticorps anti-récepteur.

### EXEMPLE 5 : Expression du fragment d'ADN codant pour la sous-unité Tbp2 de la souche IM2394 (construction avec la séquence signal homologue).

### 5A. Construction du vecteur d'expression pTG4710.

A partir du plasmide pTG3749, on a généré par PCR, à l'aide des amorces OTG4247 et OTG4248 un fragment codant pour la partie C-terminale de Tbp2 (à partir du site *BamHI* interne) et contenant un site de restriction *HindIII* en aval du codon de terminaison de traduction de *tbp2*.

Le fragment généré par PCR a été digéré par *HindIII* et *BamHI* et inséré simultanément avec le fragment *SphI* - *BamHI* de pTG3749 codant pour la partie N-terminale de Tbp2 mature, dans le vecteur pTG3743 digéré par *SphI* et *HindIII*, pour donner le plasmide pTG3786. La séquence du fragment amplifié par PCR a été vérifiée.

A partir du plasmide pTG3791, on a généré par PCR à l'aide des amorces OTG4491 et OTG4494, un fragment codant pour la partie N-terminale du précurseur de Tbp2 jusqu'au site *EcoRV* interne.

Le fragment généré par PCR a ensuite été digéré par *BspHI* et *EcoRV* et ligué simultanément aux fragments *NcoI - SstI* de pTG3704, contenant le gène *araC* et le promoteur *araB*, et *EcoRV - SstI* de pTG3786, contenant la partie 3' du gène *tbp2* et le terminateur *araB*. Le plasmide résultant pTG4710 a été vérifié par séquençage (séquence du fragment amplifié par PCR).

### 5B. Production de la sous-unité Tbp2.

*E. coli* Xac-I (Normanly et al., Proc. Natl. Acad. Sci. (1986) 83 : 6548) est transformée par le plasmide pTG4710 puis mise en culture à 37°C en milieu M9 + succinate 0,5 % + arginine 50 µg/ml + ampicilline 100 µg/ml. En phase exponentielle, on ajoute 0,2 % d'arabinose. Après différents temps d'induction (1h à 3h), on prélève des cellules et des extraits sont préparés. Une analyse en Western blot suivie d'une révélation de Tbp2 par la transferrine-peroxydase a permis de montrer que la majorité de Tbp2 se trouve sous forme de précurseur. L'analyse des extraits en SDS-PAGE suivi d'une coloration des protéines au bleu de Coomassie a permis de révéler une production importante de la protéine (évaluée à environ 5 à 10% des protéines totales). Des expériences de marquage *in vivo* au Palmitate et au glycérol tritiés ont permis de montrer que seule la forme mature est lipidée.

### EXEMPLE 6 : Expression du fragment d'ADN codant pour la sous-unité Tbp2 de la souche IM2394 (construction avec la séquence signal rlp B).

### 6A. Construction du vecteur d'expression pTG4764.

A partir de pTG3786 on a généré par PCR à l'aide des amorces OTG4494 et OTG4651, un fragment codant pour le peptide signal RlpB (Takase et al., J. Bacteriol. (1987) 169 : 5692) et le début de la séquence codant pour Tbp2 mature jusqu'au site *EcoRV* interne.
OTG4494 : cf exemple 1.

Le fragment PCR a ensuite été digéré par *BspHI* et *EcoRV* et inséré simultanément avec le fragment *EcoRV* - *HindIII* de pTG3786 porteur de la partie 3' du gène *tbp2*, dans le vecteur pTG3704 digéré par *NcoI* et *HindIII*, pour générer le plasmide pTG4764. La séquence du fragment amplifié par PCR a été vérifiée.

### 6B. Production de la sous-unité Tbp2.

*E. coli* Xac-I est transformée par le plasmide pTG4764 puis mise en culture 37°C en milieu M9 + succinate 0,5 % + arginine 50 µg/ml + ampicilline 100 µg/ml. En phase exponentielle, on ajoute 0,2 % d'arabinose. Après différents temps d'induction (1h à 3h), on prélève des cellules et des extraits sont préparés. Une analyse en Western blot suivie d'une révélation par la transferrine peroxydase a permis de détecter une bande majoritaire dont le poids moléculaire correspond à celui de Tbp2 mature purifié. La protéine est détectée dans les extraits après SDS-PAGE et coloration des protéines au bleu de Coomassie (niveau de production évalué à environ 2 à 5 % des protéines totales). Des expériences de marquage *in vivo* au palmitate et au glycérol tritiés ont permis de montrer que la protéine ainsi produite est lipidée. La quantité de forme Tbp2 mature lipidée produite à partir de la souche Xac-I/pTG4764 est supérieure à celle produite par la souche Xac-I/pTG4710.

## Revendications

1. Un fragment d'ADN isolé qui a une séquence nucléotidique codant pour une protéine ayant une 'séquence d'acides aminés qui présente un degré d'identité d'au moins 75% avec celle telle que montrée :
- dans le SEQ ID NO : 1, commençant avec le résidu cystéine en position 1 et finissant avec le résidu glutamine en position 579 ; ou
- dans le SEQ ID NO : 7, commençant avec le résidu cystéine en position 1 et finissant avec le résidu glutamine en position 691 ;
le produit d'expression de ce fragment étant reconnu par un antisérum anti-récepteur de la transferrine de la souche de *N. meningitidis* IM2394 ou IM2169.

2. Un fragment d'ADN selon la revendication 1, qui a une séquence nuctéotidique codant pour :
i) la sous-unité Tbp2 de la souche IM2394 dont la séquence en acides aminés est montrée dans le SEQ ID NO : 1, commençant avec le résidu cystéine en position 1 et finissant avec le résidu glutamine en position 579 ; ou
ii) la sous-unité Tbp2 de la souche IM2169 dont la séquence en acides aminés est montrée dans le SEQ ID NO : 7, commençant avec le résidu cystéine en position 1 et finissant avec le résidu glutamine en position 691.

3. Un fragment d'ADN selon la revendication 1, qui a une séquence nucléotidique codant pour un précurseur ayant une séquence d'acides aminés qui présente un degré d'identité d'au moins 75% avec celle telle que montrée :
- dans le SEQ ID NO : 1, commençant avec le résidu méthionine en position -20 et finissant avec le résidu glutamine en position 579 ; ou
- dans le SEQ ID NO : 7, commençant avec le résidu méthionine en position -20 et finissant avec le résidu glutamine en position 691 ;
et capable d'être reconnue par un antisérum anti-récepteur de la transferrine de la souche de *N. meningitidis* IM 2394 ou IM 2169.

4. Un fragment d'ADN selon la revendication 3, qui a une séquence nucléotidique codant pour :
i) le précurseur de la sous-unité Tbp2 de la souche IM2394 dont la séquence en acides aminés est montrée dans le SEQ ID NO : 1, commençant avec le résidu méthionine en position -20 et finissant avec le résidu glutamine en position 579 ; ou
ii) le précurseur de la sous-unité Tbp2 de la souche IM2169 dont la séquence en acides aminés est montrée dans le SEQ ID NO: 7, commençant avec le résidu méthionine en position -20 et finissant avec le résidu glutamine en position 691.

5. Un fragment d'ADN selon l'une quelconque des revendications 1 à 4, qui a une séquence d'acides aminés présentant un degré d'identité d'au moins 80% avec la séquence de référence.

6. Un fragment d'ADN selon l'une quelconque des revendications 1 à 4, qui a une séquence d'acides aminés présentant un degré d'identité d'au moins 90% avec la séquence de référence.

7. Une cassette d'expression destinée à la production d'un peptide, d'un polypeptide ou d'une protéine capable d'être reconnue par un antisérum anti-récepteur de la transferrine de la souche de *N. meningitidis* IM2394 ou IM2169, qui comprend un fragment d'ADN selon l'une des revendications 1 à 6, placé sous le contrôle des éléments nécessaires à son expression.

8. Une cassette d'expression selon la revendication 7, dans laquelle ledit fragment d'ADN est placé sous le contrôle d'éléments comprenant le promoteur *araB* de *S. typhimurium*.

9. Une cassette d'expression selon la revendication 7 ou 8, destinée à la production d'une protéine ayant une séquence d'acides aminés qui présente un degré d'identité d'au moins 75% avec celle telle que montrée :
- dans le SEQ ID NO : 7, commençant avec le résidu cystéine en position 1 et finissant avec le résidu glutamine en position 691 ;
qui comprend :
i) un bloc d'ADN codant pour le peptide signal RipB et
ii) un fragment d'ADN codant pour ladite protéine.

10. Une cassette d'expression selon l'une quelconque des revendications 7 à 9, destinée à la production d'une protéine ayant une séquence d'acides aminés qui présente un degré d'identité d'au moins 80% avec la séquence en référence.

11. Une cassette d'expression selon l'une quelconque des revendications 7 à 9, destinée à la production d'une protéine ayant une séquence d'acides aminés qui présente un degré d'identité d'au moins 90% avec la séquence de référence.

12. Une cassette d'expression selon la revendication 9, destinée à la production de la sous-unité Tbp2 de *N. meningitidis* IM2394 ou IM2169, qui comprend :
i) un bloc d'ADN codant pour le peptide signal RipB et
ii) un fragment d'ADN codant pour ladite sous-unité.

13. Une cellule-hôte transformée par une cassette d'expression selon l'une des revendications 7 à 12.

14. Un procédé de production d'un peptide, d'un polypeptide ou d'une protéine capable d'être reconnue par un antisérum anti-récepteur de la transferrine de la souche de *N. meningitidis* IM2394 ou IM2169, selon lequel on cultive une cellule-hôte selon la revendication 13 et on recueille ledit peptide, ledit polypeptide ou ladite protéine à partir de la culture.

15. Une composition pharmaceutique comprenant à titre de principe actif, un peptide, un polypeptide ou une protéine capable d'être reconnue par un antisérum anti-récepteur de la transferrine de la souche de *N. meningitidis* IM2394 ou IM2169, obtenue par le procédé selon la revendication 14.

16. Une composition pharmaceutique comprenant à titre de principe actif, un vecteur viral ou bactérien dans le génome duquel est inséré un fragment d'ADN selon l'une des revendications 1 à 6, placé sous le contrôle des éléments nécessaires à son expression.

17. Un bloc d'ADN isolé codant pour un peptide signal ayant une séquence d'acides aminés qui présente un degré d'identité d'au moins 75% avec celle telle que montrée dans :
- le SEQ ID NO : 7, commençant avec le résidu méthionine en position -20 et finissant avec le résidu alanine en position -1 ;
et capable d'être reconnue par un antisérum anti-récepteur de la transferrine de la souche de *N. meningitidis* IM2394 ou IM 2169.

18. Un bloc d'ADN isolé codant pour un peptide signal ayant une séquence d'acides aminés telle que montrée dans :
- le SEQ ID NO: 7, commençant avec le résidu méthionine en position -20 et finissant avec le résidu alanine en position -1.

19. Un bloc d'ADN selon la revendication 17, codant pour un peptide signal ayant une séquence d'acides aminés présentant un degré d'identité d'au moins 80% avec la séquence de référence.

20. Un bloc d'ADN selon la revendication 17, codant pour un peptide signal ayant une séquence d'acides aminés présentant un degré d'identité d'au moins 90% avec la séquence de référence.

## Claims

1. An isolated DNA fragment having a nucleotide sequence coding for a protein having an amino acid sequence having a degree of identity of at least 75% with that such as is demonstrated:
- in the SEQ ID No. 1, commencing with the cysteine residue in the 1 position and finishing with the glutamine residue in the 579 position, or
- in the SEQ ID No. 7, commencing with the cysteine residue in the 1 position and finishing with the glutamine residue in the 691 position,
the expression product of said fragment being recognized by an antireceptor antiserum of transferrin of the N. meningitidis IM2394 or IM2169 strain.

2. A DNA fragment according to claim 1 having a nucleotide sequence coding for:
i) the Tbp2 subunit of the IM2394 strain, whose amino acid sequence is demonstrated in SEQ ID No. 1, commencing with the cysteine residue in the 1 position and finishing with the glutamine residue in the 579 position, or
ii) the Tbp2 subunit of the IM2169 strain, whose amino acid sequence is demonstrated in the SEQ ID No. 7, commencing with the cysteine residue in the 1 position and finishing with the glutamine residue in the 691 position.

3. A DNA fragment according to claim 1 having a nucleotide sequence coding for a precursor having an amino acid sequence with a degree of identity of at least 75% with that such as demonstrated:
- in the SEQ ID No. 1, commencing with the methionine residue in the -20 position and finishing with the glutamine residue in the 579 position, or
- in the SEQ ID No. 7, commencing with the methionine residue in the -20 position and finishing with the glutamine residue in the 691 position,
and which can be recognized by an antireceptor antiserum of transferrin of the IM 2394 or IM 2169 N. meningitidis stain.

4. A DNA fragment according to claim 3 having a nucleotide sequence coding for:
i) the precursor of the Tbp2 subunit of the IM2394 strain, whose amino acid sequence is demonstrated in SEQ ID No. 1, commencing with the methionine residue in the -20 position and finishing with the glutamine residue in the 579 position, or
ii) the precursor of the Tbp2 subunit of the IM2169 strain, whose amino acid sequence is demonstrated in SEQ ID No. 7, commencing with the methionine residue in the -20 position and finishing with the glutamine residue in the 691 position.

5. A DNA fragment according to any one of the claims 1 to 4 having an amino acid sequence with a degree of identity of at least 80% with the reference sequence.

6. A DNA fragment according to any one of the claims 1 to 4 having an amino acid sequence with a degree of identity of at least 90% with the reference sequence.

7. An expression cassette intended for the production of a peptide, a polypeptide or a protein recognizable by an antireceptor antiserum of transferrin of the IM2394 or IM2169 N. meningitidis strain comprising a DNA fragment according to one of the claims 1 to 6, placed under the control of the elements necessary for its expression.

8. An expression cassette according to claim 7, wherein said DNA fragment is placed under the control of elements comprising the S. typhimurium araB promoter.

9. An expression cassette according to claim 7 or 8 intended for the production of a protein having an amino acid sequence with a degree of identity of at least 75% with that such as is demonstrated:
- in SEQ ID No. 7, commencing with the cysteine residue in the 1 position and finishing with the glutamine residue in the 691 position,
which comprises:
i) a DNA block coding for the signal peptide RipB and
ii) a DNA fragment coding for said protein.

10. An expression cassette according to any one of the claims 7 to 9 intended for the production of a protein having an amino acid sequence with a degree of identity of at least 80% with the reference sequence.

11. An expression cassette according to any one of the claims 7 to 9 intended for the production of a protein having an amino acid sequence with a degree of identity of at least 90% with the reference sequence.

12. An expression cassette according to claim 9 intended for the production of the IM2394 or IM2169 N. meningitidis Tbp2 subunit and which comprises:
i) a DNA block coding for the signal peptide RipB and
ii) a DNA fragment coding for said subunit.

13. A host cell transformed by an expression cassette according to one of the claims 7 to 12.

14. A process for the production of a peptide, a polypeptide or protein which can be recognized by an antireceptor antiserum of transferrin of the IM2394 or IM2169 N. meningitidis strain, according to which a host cell according to claim 13 is cultured and said peptide, said polypeptide or said protein is harvested from the culture.

15. A pharmaceutical composition comprising as the active principle a peptide, a polypeptide or a protein which can be recognized by an antireceptor antiserum of transferring of the IM2394 or IM2169 N. meningitidis strain obtained by the process according to claim 14.

16. A pharmaceutical composition comprising as the active principle a viral or bacterial vector in the genome of which is inserted a DNA fragment according to one of the claims 1 to 6, placed under the control of the elements necessary for its expression.

17. An isolated DNA block coding for a signal peptide having an amino acid sequence with a degree of identity of at least 75% with that such as is demonstrated in:
- the SEQ ID no. 7, commencing with the methionine residue in the -20 position and finishing with the alanine residue in the -1 position,
- and which can be recognized by an antireceptor antiserum of transferrin of the IM2394 or IM2169 N. meningitidis strain.

18. An isolated DNA block coding for a signal peptide having an amino acid sequence as demonstrated in:
- the SEQ ID no. 7, commencing with the methionine residue in the -20 position and finishing with the alanine residue in the -1 position.

19. A DNA block according to claim 17, coding for a signal peptide having an amino acid sequence with a degree of identity of at least 80% with the reference sequence.

20. A DNA block according to claim 17, coding for a signal peptide having an amino acid sequence with a degree of identity of at least 90% with the reference sequence.

## Patentansprüche

1. Isoliertes DNA-Fragment, das eine Nucleotidsequenz besitzt, die für ein Protein codiert, das eine Aminosäuresequenz hat, die einen Grad der Identität von mindestens 75 % mit derjenigen, die wie
- in der SEQ ID Nr. 1, die mit dem Cysteinrest in Position 1 beginnt und mit dem Glutaminrest in Position 579 endet, oder
- in der SEQ ID Nr. 7, die mit dem Cysteinrest in Position 1 beginnt und mit dem Glutaminrest in Position 691 endet,
gezeigt ist, aufweist, wobei das Expressionsprodukt dieses Fragments von einem gegen den Transferrinrezeptor des Stamms *N. Meningitidis* IM2394 oder IM2169 gerichteten Antiserum erkannt wird.

2. DNA-Fragment nach Anspruch 1, das eine Nucleotidsequenz besitzt, die codiert für
I) die Untereinheit Tbp2 des Stamms IM2394, deren Aminosäuresequenz in der SEQ ID Nr. 1, die mit dem Cysteinrest in Position 1 beginnt und mit dem Glutaminrest in Position 579 endet, oder
II) die Untereinheit Tbp2 des Stamms IM2169, deren Aminosäuresequenz in der SEQ ID Nr. 7, die mit dem Cysteinrest in Position 1 beginnt und mit dem Glutaminrest in Position 691 endet, gezeigt ist.

3. DNA-Fragment nach Anspruch 1, das eine Nucleotidsequenz besitzt, die für einen Vorläufer mit einer Aminosäuresequenz codiert, die einen Grad der Identität von mindestens 75 % mit derjenigen, die wie
- in der SEQ ID Nr. 1, die mit dem Methioninrest in Position -20 beginnt und mit dem Glutaminrest in Position 579 endet, oder
- in der SEQ ID Nr. 7, die mit dem Methioninrest in Position -20 beginnt und mit dem Glutaminrest in Position 691 endet,
gezeigt ist, aufweist und in der Lage ist, von einem gegen den Transferrinrezeptor des Stamms von *N. Meningitidis* IM 2394 oder IM 2169 gerichteten Antiserum erkannt zu werden.

4. DNA-Fragment nach Anspruch 3, das eine Nucleotidsequenz besitzt, die codiert für
I) den Vorläufer der Untereinheit Tbp2 des Stamms IM2394, deren Aminosäuresequenz in der SEQ ID Nr. 1, die mit dem Methioninrest in Position -20 beginnt und mit dem Glutaminrest in Position 579 endet, gezeigt ist, oder
II) den Vorläufer der Untereinheit Tbp2 des Stamms IM2169, deren Aminosäuresequenz wie in der SEQ ID Nr. 7, die mit dem Methioninrest in Position -20 beginnt und mit dem Glutaminrest in Position 691 endet, gezeigt ist.

5. DNA-Fragment nach einem der Ansprüche 1 bis 4, das eine Aminosäuresequenz besitzt, die einen Identitätsgrad von mindestens 80 % mit der Referenzsequenz aufweist.

6. DNA-Fragment nach einem der Ansprüche 1 bis 4, das eine Aminosäuresequenz besitzt, die einen Identitätsgrad von mindestens 90 % mit der Referenzsequenz aufweist.

7. Expressionskassette, die für die Produktion eines Peptids, eines Polypeptids oder eines Proteins, das in der Lage ist, von einem am Transferrinrezeptor des Stamms von *N. Meningitidis* IM2394 oder IM2169 antagonistischen Antiserum erkannt zu werden, vorgesehen ist und ein DNA-Fragment nach einem der Ansprüche 1 bis 6, das unter der Kontrolle der für seine Expression erforderlichen Elemente angeordnet worden ist, enthält.

8. Expressionskassette nach Anspruch 7, in welcher das DNA-Fragment unter der Kontrolle von Elementen angeordnet wird, die den Promotor araB von *S. Typhimurium* enthalten.

9. Expressionskassette nach Anspruch 7 oder 8, die für die Produktion eines Proteins mit einer Aminosäuresequenz vorgesehen ist, die einen Grad der Identität von mindestens 75 % mit derjenigen aufweist, die wie
- in der SEQ ID Nr. 7, die mit dem Cysteinrest in Position 1 beginnt und mit dem Glutaminrest in Position 691 endet, gezeigt ist,
und welche
I) einen DNA-Block, der für das Signalpeptid RipB codiert, und
II) ein für dieses Protein codierendes DNA-Fragment
umfasst.

10. Expressionskassette nach einem der Ansprüche 7 bis 9, die für die Produktion eines Proteins mit einer Aminosäuresequenz vorgesehen ist, die einen Identitätsgrad von mindestens 80 % mit der Referenzsequenz aufweist.

11. Expressionskassette nach einem der Ansprüche 7 bis 9, die für die Produktion eines Proteins mit einer Aminosäuresequenz vorgesehen ist, die einen Identitätsgrad von mindestens 90 % mit der Referenzsequenz aufweist.

12. Expressionskassette nach Anspruch 9, die für die Produktion der Untereinheit Tbp2 von *N. Meningitidis* IM2394 oder IM2169 vorgesehen ist und
I) einen DNA-Block, der für das Signalpeptid RipB codiert, und
II) ein für diese Untereinheit codierendes DNA-Fragment
umfasst.

13. Wirtszelle, die von einer Expressionskassette nach einem der Ansprüche 7 bis 12 transformiert worden ist.

14. Verfahren zur Herstellung eines Peptids, Polypeptids oder Proteins, das in der Lage ist, von einem gegen den Transferrinrezeptor des Stamms von *N. Meningitidis* IM2394 oder IM2169 gerichteten Antiserum erkannt zu werden, gemäß welchem eine Wirtszelle nach Anspruch 13 kultiviert und das Peptid, Polypeptid oder Protein aus der Kultur gewonnen wird.

15. Pharmazeutische Zusammensetzung, die als Wirkstoff ein Peptid, Polypeptid oder Protein enthält, das in der Lage ist, von einem gegen den Transferrinrezeptor des Stamms von *N. Meningitidis* IM2394 oder IM2169 gerichteten Antiserum erkannt zu werden und welches durch das Verfahren nach Anspruch 14 erhalten worden ist.

16. Pharmazeutische Zusammensetzung, die als Wirkstoff einen viralen oder bakteriellen Vektor enthält, in dessen Genom ein DNA-Fragment nach einem der Ansprüche 1 bis 6 insertiert ist, das unter der Kontrolle der für seine Expression erforderlichen Elemente angeordnet worden ist.

17. Isolierter DNA-Block, der für ein Signalpeptid mit einer Aminosäuresequenz codiert, die einen Grad der Identität von mindestens 75 % mit derjenigen, die wie in
- SEQ ID Nr. 7, die in Position -20 mit dem Methioninrest beginnt und in Position -1 mit dem Alaninrest endet, gezeigt ist,
aufweist und in der Lage ist, von einem gegen den Transferrinrezeptor des Stamms von *N. Meningitidis* IM2394 oder IM2169 gerichteten Antiserum erkannt zu werden.

18. Isolierter DNA-Block, der für ein Signalpeptid mit einer Aminosäuresequenz wie derjenigen codiert, die in
- SEQ ID Nr. 7, die mit dem Methioninrest in Position -20 beginnt und mit dem Alaninrest in Position -1 endet, gezeigt ist.

19. DNA-Block nach Anspruch 17, der für ein Signalpeptid mit einer Aminosäuresequenz codiert, die einen Identitätsgrad von mindestens 80 % mit der Referenzsequenz aufweist.

20. DNA-Block nach Anspruch 17, der für ein Signalpeptid mit einer Aminosäuresequenz codiert, die einen Identitätsgrad von mindestens 90 % mit der Referenzsequenz aufweist.


### LISTE DE SEQUENCES

(1) INFORMATION GENERALE:
   (i) DEPOSANT:
      (A) NOM: Pasteur Mérieux Sérums & Vaccins
      (B) RUE: 58, avenue Leclerc
      (C) VILLE: Lyon
      (E) PAYS: France
      (F) CODE POSTAL: 69007

      (A) NOM: Transgène
      (B) RUE: 11, rue de Molsheim
      (C) VILLE: Strasbourg
      (E) PAYS: France
      (F) CODE POSTAL: 67000
   (ii) TITRE DE L' INVENTION: Fragments d'ADN codant pour les sous-unites du recepteur de la transferrine de Neisseria meningitidis et procede les exprimant.
   (iii) NOMBRE DE SEQUENCES: 24
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (v) DONNEES DE LA DEMANDE ACTUELLE :
      NUMERO DE DEPOT: EP 93401531.4
   (vi) DONNEES DE LA DEMANDE ANTERIEURE:
      (A) NUMERO DE DEPOT: FR 92 07493
      (B) DATE DE DEPOT: 19-JUN-1992
(2) INFORMATION POUR LA SEQ ID NO: 1:
   (i) CARACTERISTIQUES DE LA SEQUENCE:
      (A) LONGUEUR: 1808 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Dna codant pour la sous-unite Tbp2 du recepteur transferrine
      (B) SOUCHE: Neisseria meningitidis IM2394
   (ix) CARACTERISTIQUE ADDITIONELLE:
      (A) NOM/CLE: sig peptide
      (B) EMPLACEMENT: 1..60
   (ix) CARACTERISTIQUE ADDITIONELLE:
      (A) NOM/CLE: mat peptide
      (B) EMPLACEMENT: 61..1797
   (ix) CARACTERISTIQUE ADDITIONELLE:
      (A) NOM/CLE: CDS
      (B) EMPLACEMENT: 1..1797
   (xi) DESCRIPTION DE LA SEQUENCE: SEQ ID NO: 1:
   ATG AAC AAT CCA TTG GTA AAT CAG GCT GCT ATG GTG CTG CCT GTG TTT      48
   Met Asn Asn Pro Leu Val Asn Gln Ala Ala Met Val Leu Pro Val Phe
   -20                 -15                 -10                  -5
   TTG TTG AGT GCT TGT CTG GGT GGC GGC GGC AGT TTC GAT TTG GAC AGC      96
   Leu Leu Ser Ala Cys Leu Gly Gly Gly Gly Ser Phe Asp Leu Asp Ser
                     1               5                  10
   GTG GAA ACC GTG CAA GAT ATG CAC TCC AAA CCT AAG TAT GAG GAT GAA     144
   Val Glu Thr Val Gln Asp Met His Ser Lys Pro Lys Tyr Glu Asp Glu
            15                  20                  25
   AAA AGC CAG CCT GAA AGC CAA CAG GAT GTA TCG GAA AAC AGC GGC GCG     192
   Lys Ser Gln Pro Glu Ser Gln Gln Asp Val Ser Glu Asn Ser Gly Ala
        30                  35                  40
   GCT TAT GGC TTT GCA GTA AAA CTA CCT CGC CGG AAT GCA CAT TTT AAT     240
   Ala Tyr Gly Phe Ala Val Lys Leu Pro Arg Arg Asn Ala His Phe Asn
    45                  50                  55                  60
   CCT AAA TAT AAG GAA AAG CAC AAA CCA TTG GGT TCA ATG GAT TGG AAA     288
   Pro Lys Tyr Lys Glu Lys His Lys Pro Leu Gly Ser Met Asp Trp Lys
                    65                  70                  75
   AAA CTG CAA AGA GGA GAA CCA AAT AGT TTT AGT GAG AGG GAT GAA TTG     336
   Lys Leu Gln Arg Gly Glu Pro Asn Ser Phe Ser Glu Arg Asp Glu Leu
                80                  85                  90
   GAA AAA AAA CGG GGT AGT TCT GAA CTT ATT GAA TCA AAA TGG GAA GAT     384
   Glu Lys Lys Arg Gly Ser Ser Glu Leu Ile Glu Ser Lys Trp Glu Asp
            95                 100                 105
   GGG CAA AGT CGT GTA GTT GGT TAT ACA AAT TTC ACT TAT GTC CGT TCG     432
   Gly Gln Ser Arg Val Val Gly Tyr Thr Asn Phe Thr Tyr Val Arg Ser
       110                 115                 120
   GGA TAT GTT TAC CTT AAT AAA AAT AAT ATT GAT ATT AAG AAT AAT ATA      480
   Gly Tyr Val Tyr Leu Asn Lys Asn Asn Ile Asp Ile Lys Asn Asn Ile
   125                 130                 135                 140
   GTT CTT TTT GGA CCT GAC GGA TAT CTT TAC TAT AAA GGG AAA GAA CCT      528
   Val Leu Phe Gly Pro Asp Gly Tyr Leu Tyr Tyr Lys Gly Lys Glu Pro
                   145                 150                 155
   TCC AAG GAG CTG CCA TCG GAA AAG ATA ACT TAT AAA GGT ACT TGG GAT      576
   Ser Lys Glu Leu Pro Ser Glu Lys Ile Thr Tyr Lys Gly Thr Trp Asp
               160                 165                 170
   TAT GTT ACT GAT GCT ATG GAA AAA CAA AGG TTT GAA GGA TTG GGT AGT      624
   Tyr Val Thr Asp Ala Met Glu Lys Gln Arg Phe Glu Gly Leu Gly Ser
           175                 180                 185
   GCA GCA GGA GGA GAT AAA TCG GGG GCG TTG TCT GCA TTA GAA GAA GGG      672
   Ala Ala Gly Gly Asp Lys Ser Gly Ala Leu Ser Ala Leu Glu Glu Gly
       190                 195                 200
   GTA TTG CGT AAT CAG GCA GAG GCA TCA TCC GGT CAT ACC GAT TTT GGT      720
   Val Leu Arg Asn Gln Ala Glu Ala Ser Ser Gly His Thr Asp Phe Gly
   205                 210                 215                 220
   ATG ACT AGT GAG TTT GAG GTT GAT TTT TCT GAT AAA ACA ATA AAG GGC      768
   Met Thr Ser Glu Phe Glu Val Asp Phe Ser Asp Lys Thr Ile Lys Gly
                   225                 230                 235
   ACA CTT TAT CGT AAC AAC CGT ATT ACT CAA AAT AAT AGT GAA AAC AAA      816
   Thr Leu Tyr Arg Asn Asn Arg Ile Thr Gln Asn Asn Ser Glu Asn Lys
               240                 245                 250
   CAA ATA AAA ACT ACG CGT TAC ACC ATT CAA GCA ACT CTT CAC GGC AAC      864
   Gln Ile Lys Thr Thr Arg Tyr Thr Ile Gln Ala Thr Leu His Gly Asn
           255                 260                 265
   CGT TTC AAA GGT AAG GCG TTG GCG GCA GAT AAA GGT GCA ACA AAT GGA      912
   Arg Phe Lys Gly Lys Ala Leu Ala Ala Asp Lys Gly Ala Thr Asn Gly
       270                 275                 280
   AGT CAT CCC TTT ATT TCC GAC TCC GAC AGT TTG GAA GGC GGA TTT TAC      960
   Ser His Pro Phe Ile Ser Asp Ser Asp Ser Leu Glu Gly Gly Phe Tyr
   285                 290                 295                 300
   GGG CCG AAA GGC GAG GAA CTT GCC GGT AAA TTC TTG AGC AAC GAC AAC     1008
   Gly Pro Lys Gly Glu Glu Leu Ala Gly Lys Phe Leu Ser Asn Asp Asn
                   305                 310                 315
   AAA GTT GCA GCG GTG TTT GGT GCG AAG CAG AAA GAT AAG AAG GAT GGG     1056
   Lys Val Ala Ala Val Phe Gly Ala Lys Gln Lys Asp Lys Lys Asp Gly
               320                 325                 330
   GAA AAC GCG GCA GGG CCT GCA ACG GAA ACC GTG ATA GAT GCA TAC CGT      1104
   Glu Asn Ala Ala Gly Pro Ala Thr Glu Thr Val Ile Asp Ala Tyr Arg
           335                 340                 345
   ATT ACC GGC GAG GAG TTT AAG AAA GAG CAA ATA GAC AGT TTT GGA GAT      1152
   Ile Thr Gly Glu Glu Phe Lys Lys Glu Gln Ile Asp Ser Phe Gly Asp
       350                 355                 360
   GTG AAA AAG CTG CTG GTT GAC GGA GTG GAG CTT TCA CTG CTG CCG TCT      1200
   Val Lys Lys Leu Leu Val Asp Gly Val Glu Leu Ser Leu Leu Pro Ser
   365                 370                 375                 380
   GAG GGC AAT AAG GCG GCA TTT CAG CAC GAG ATT GAG CAA AAC GGC GTG      1248
   Glu Gly Asn Lys Ala Ala Phe Gln His Glu Ile Glu Gln Asn Gly Val
                   385                 390                 395
   AAG GCA ACG GTG TGT TGT TCC AAC TTG GAT TAC ATG AGT TTT GGG AAG      1296
   Lys Ala Thr Val Cys Cys Ser Asn Leu Asp Tyr Met Ser Phe Gly Lys
               400                 405                 410
   CTG TCA AAA GAA AAT AAA GAC GAT ATG TTC CTG CAA GGT GTC CGC ACT      1344
   Leu Ser Lys Glu Asn Lys Asp Asp Met Phe Leu Gln Gly Val Arg Thr
           415                 420                 425
   CCA GTA TCC GAT GTG GCG GCA AGG ACG GAG GCA AAC GCC AAA TAT CGC      1392
   Pro Val Ser Asp Val Ala Ala Arg Thr Glu Ala Asn Ala Lys Tyr Arg
       430                 435                 440
   GGT ACT TGG TAC GGA TAT ATT GCC AAC GGC ACA AGC TGG AGC GGC GAA      1440
   Gly Thr Trp Tyr Gly Tyr Ile Ala Asn Gly Thr Ser Trp Ser Gly Glu
   445                 450                 455                 460
   GCC TCC AAT CAG GAA GGT GGT AAT AGG GCA GAG TTT GAC GTG GAT TTT      1488
   Ala Ser Asn Gln Glu Gly Gly Asn Arg Ala Glu Phe Asp Val Asp Phe
                   465                 470                 475
   TCC ACT AAA AAA ATC AGT GGC ACA CTG ACG GCA AAA GAC CGT ACG TCT      1536
   Ser Thr Lys Lys Ile Ser Gly Thr Leu Thr Ala Lys Asp Arg Thr Ser
               480                 485                 490
   CCT GCG TTT ACT ATT ACT GCC ATG ATT AAG GAC AAC GGT TTT TCA GGT      1584
   Pro Ala Phe Thr Ile Thr Ala Met Ile Lys Asp Asn Gly Phe Ser Gly
           495                 500                 505
   GTG GCG AAA ACC GGT GAA AAC GGC TTT GCG CTG GAT CCG CAA AAT ACC      1632
   Val Ala Lys Thr Gly Glu Asn Gly Phe Ala Leu Asp Pro Gln Asn Thr
       510                 515                 520
   GGA AAT TCC CAC TAT ACG CAT ATT GAA GCC ACT GTA TCC GGC GGT TTC      1680
   Gly Asn Ser His Tyr Thr His Ile Glu Ala Thr Val Ser Gly Gly Phe
   525                 530                 535                 540
   TAC GGC AAA AAC GCC ATC GAG ATG GGC GGA TCG TTC TCA TTT CCG GGA      1728
   Tyr Gly Lys Asn Ala Ile Glu Met Gly Gly Ser Phe Ser Phe Pro Gly
                   545                 550                 555
   AAT GCA CCA GAG GGA AAA CAA GAA AAA GCA TCG GTG GTA TTC GGT GCG      1776
   Asn Ala Pro Glu Gly Lys Gln Glu Lys Ala Ser Val Val Phe Gly Ala
               560                 565                 570
   AAA CGC CAA CAG CTT GTG CAA TAAGCACGGC T                             1808
   Lys Arg Gln Gln Leu Val Gln
           575
(2) INFORMATION POUR LA SEQ ID NO: 2:
   (i) CARACTERISTIQUES DE LA SEQUENCE:
      (A) LONGUEUR: 599 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Asn Asn Pro Leu Val Asn Gln Ala Ala Met Val Leu Pro Val Phe
   -20                 -15                 -10                  -5
   Leu Leu Ser Ala Cys Leu Gly Gly Gly Gly Ser Phe Asp Leu Asp Ser
                        1               5                  10
   Val Glu Thr Val Gln Asp Met His Ser Lys Pro Lys Tyr Glu Asp Glu
           15                  20                  25
   Lys Ser Gln Pro Glu Ser Gln Gln Asp Val Ser Glu Asn Ser Gly Ala
       30                  35                  40
   Ala Tyr Gly Phe Ala Val Lys Leu Pro Arg Arg Asn Ala His Phe Asn
   45                  50                  55                  60
   Pro Lys Tyr Lys Glu Lys His Lys Pro Leu Gly Ser Met Asp Trp Lys
                   65                  70                  75
   Lys Leu Gln Arg Gly Glu Pro Asn Ser Phe Ser Glu Arg Asp Glu Leu
               80                  85                  90
   Glu Lys Lys Arg Gly Ser Ser Glu Leu Ile Glu Ser Lys Trp Glu Asp
           95                 100                 105
   Gly Gln Ser Arg Val Val Gly Tyr Thr Asn Phe Thr Tyr Val Arg Ser
       110                 115                 120
   Gly Tyr Val Tyr Leu Asn Lys Asn Asn Ile Asp Ile Lys Asn Asn Ile
   125                 130                 135                 140
   Val Leu Phe Gly Pro Asp Gly Tyr Leu Tyr Tyr Lys Gly Lys Glu Pro
   145 150 155
   Ser Lys Glu Leu Pro Ser Glu Lys Ile Thr Tyr Lys Gly Thr Trp Asp
   160 165 170
   Tyr Val Thr Asp Ala Met Glu Lys Gln Arg Phe Glu Gly Leu Gly Ser
   175 180 185
   Ala Ala Gly Gly Asp Lys Ser Gly Ala Leu Ser Ala Leu Glu Glu Gly
   190 195 200
   Val Leu Arg Asn Gln Ala Glu Ala Ser Ser Gly His Thr Asp Phe Gly
   205 210 215 220
   Met Thr Ser Glu Phe Glu Val Asp Phe Ser Asp Lys Thr Ile Lys Gly
   225 230 235
   Thr Leu Tyr Arg Asn Asn Arg Ile Thr Gln Asn Asn Ser Glu Asn Lys
   240 245 250
   Gln Ile Lys Thr Thr Arg Tyr Thr Ile Gln Ala Thr Leu His Gly Asn
   255 260 265
   Arg Phe Lys Gly Lys Ala Leu Ala Ala Asp Lys Gly Ala Thr Asn Gly
   270 275 280
   Ser His Pro Phe Ile Ser Asp Ser Asp Ser Leu Glu Gly Gly Phe Tyr
   285 290 295 300
   Gly Pro Lys Gly Glu Glu Leu Ala Gly Lys Phe Leu Ser Asn Asp Asn
   305 310 315
   Lys Val Ala Ala Val Phe Gly Ala Lys Gln Lys Asp Lys Lys Asp Gly
   320 325 330
   Glu Asn Ala Ala Gly Pro Ala Thr Glu Thr Val Ile Asp Ala Tyr Arg
   335 340 345
   Ile Thr Gly Glu Glu Phe Lys Lys Glu Gln Ile Asp Ser Phe Gly Asp
   350 355 360
   Val Lys Lys Leu Leu Val Asp Gly Val Glu Leu Ser Leu Leu Pro Ser
   365 370 375 380
   Glu Gly Asn Lys Ala Ala Phe Gln His Glu Ile Glu Gln Asn Gly Val
   385 390 395
   Lys Ala Thr Val Cys Cys Ser Asn Leu Asp Tyr Met Ser Phe Gly Lys
   400 405 410
   Leu Ser Lys Glu Asn Lys Asp Asp Met Phe Leu Gln Gly Val Arg Thr
   415 420 425
   Pro Val Ser Asp Val Ala Ala Arg Thr Glu Ala Asn Ala Lys Tyr Arg
   430 435 440
   Gly Thr Trp Tyr Gly Tyr Ile Ala Asn Gly Thr Ser Trp Ser Gly Glu
   445 450 455 460
   Ala Ser Asn Gln Glu Gly Gly Asn Arg Ala Glu Phe Asp Val Asp Phe
   465 470 475
   Ser Thr Lys Lys Ile Ser Gly Thr Leu Thr Ala Lys Asp Arg Thr Ser
   480 485 490
   Pro Ala Phe Thr Ile Thr Ala Met Ile Lys Asp Asn Gly Phe Ser Gly
   495 500 505
   Val Ala Lys Thr Gly Glu Asn Gly Phe Ala Leu Asp Pro Gln Asn Thr
   510 515 520
   Gly Asn Ser His Tyr Thr His Ile Glu Ala Thr Val Ser Gly Gly Phe
   525 530 535 540
   Tyr Gly Lys Asn Ala Ile Glu Met Gly Gly Ser Phe Ser Phe Pro Gly
   545 550 555
   Asn Ala Pro Glu Gly Lys Gln Glu Lys Ala Ser Val Val Phe Gly Ala
   560 565 570
   Lys Arg Gln Gln Leu Val Gln
   575
(2) INFORMATION POUR LA SEQ ID NO: 3:
   (i) CARACTERISTIQUES DE LA SEQUENCE:
      (A) LONGUEUR: 2800 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: DNA codant pour la sous-unité Tbp1 du recepteur transferrine
      (B) SOUCHE: Neisseria meningitidis IM2394
   (ix) CARACTERISTIQUE ADDITIONELLE:
      (A) NOM/CLE: sig peptide
      (B) EMPLACEMENT: 40..111
   (ix) CARACTERISTIQUE ADDITIONELLE:
      (A) NOM/CLE: mat peptide
      (B) EMPLACEMENT: 112..2763
   (ix) CARACTERISTIQUE ADDITIONELLE:
      (A) NOM/CLE: CDS
      (B) EMPLACEMENT: 40..2763
   (xi) DESCRIPTION DE LA SEQUENCE: SEQ ID NO: 3:
   CTTCCGATGC CGTCTGAAAG CGAAGATTAG GGAAACACT ATG CAA CAG CAA CAT        54
                                             Met Gln Gln Gln His
                                             -24             -20
   TTG TTC CGA TTA AAT ATT TTA TGC CTG TCT TTA ATG ACC GCG CTG CCC      102
   Leu Phe Arg Leu Asn Ile Leu Cys Leu Ser Leu Met Thr Ala Leu Pro
                   -15                 -10                  -5
   GTT TAT GCA GAA AAT GTG CAA GCC GAA CAA GCA CAG GAA AAA CAG TTG      150
   Val Tyr Ala Glu Asn Val Gln Ala Glu Gln Ala Gln Glu Lys Gln Leu
                1               5                  10
   GAT ACC ATA CAG GTA AAA GCC AAA AAA CAG AAA ACC CGC CGC GAT AAC      198
   Asp Thr Ile Gln Val Lys Ala Lys Lys Gln Lys Thr Arg Arg Asp Asn
       15                  20                  25
   GAA GTA ACC GGG CTG GGC AAG TTG GTC AAG TCT TCC GAT ACG CTA AGT      246
   Glu Val Thr Gly Leu Gly Lys Leu Val Lys Ser Ser Asp Thr Leu Ser
   30                  35                  40                  45
   AAA GAA CAG GTT TTG AAT ATC CGA GAC CTG ACC CGT TAT GAT CCG GGT      294
   Lys Glu Gln Val Leu Asn Ile Arg Asp Leu Thr Arg Tyr Asp Pro Gly
                   50                  55                  60
   ATT GCC GTG GTC GAA CAG GGT CGG GGC GCA AGT TCC GGC TAT TCA ATA      342
   Ile Ala Val Val Glu Gln Gly Arg Gly Ala Ser Ser Gly Tyr Ser Ile
               65                  70                  75
   CGC GGC ATG GAT AAA AAC CGC GTT TCC TTA ACG GTA GAC GGC GTT TCG      390
   Arg Gly Met Asp Lys Asn Arg Val Ser Leu Thr Val Asp Gly Val Ser
           80                  85                  90
   CAA ATA CAG TCC TAC ACC GCG CAG GCG GCA TTG GGT GGG ACG AGG ACG      438
   Gln Ile Gln Ser Tyr Thr Ala Gln Ala Ala Leu Gly Gly Thr Arg Thr
       95                  100                 105
   GCG GGT AGC AGC GGC GCA ATC AAT GAA ATC GAG TAT GAA AAC GTC AAG      486
   Ala Gly Ser Ser Gly Ala Ile Asn Glu Ile Glu Tyr Glu Asn Val Lys
   110                 115                 120                 125
   GCC GTT GAA ATC AGC AAG GGT TCG AAT TCA TCA GAA TAC GGA AAC GGC        534
   Ala Val Glu Ile Ser Lys Gly Ser Asn Ser Ser Glu Tyr Gly Asn Gly
                   130                 135                 140
   GCA TTG GCA GGT TCG GTC GCA TTT CAA ACC AAA ACC GCA GCC GAC ATT        582
   Ala Leu Ala Gly Ser Val Ala Phe Gln Thr Lys Thr Ala Ala Asp Ile
               145                 150                 155
   ATC GGA GAG GGA AAA CAG TGG GGC ATT CAG AGT AAA ACT GCC TAT TCG        630
   Ile Gly Glu Gly Lys Gln Trp Gly Ile Gln Ser Lys Thr Ala Tyr Ser
           160                 165                 170
   GGA AAA GAC CAT GCC CTG ACG CAA TCC CTT GCG CTT GCC GGA CGC AGC        678
   Gly Lys Asp His Ala Leu Thr Gln Ser Leu Ala Leu Ala Gly Arg Ser
       175                 180                 185
   GGC GGC GCG GAA GCC CTC CTT ATT TAT ACT AAA CGG CGG GGT CGG GAA        726
   Gly Gly Ala Glu Ala Leu Leu Ile Tyr Thr Lys Arg Arg Gly Arg Glu
   190                 195                 200                 205
   ATC CAT GCG CAT AAA GAT GCC GGC AAG GGT GTG CAG AGC TTC AAC CGG        774
   Ile His Ala His Lys Asp Ala Gly Lys Gly Val Gln Ser Phe Asn Arg
                   210                 215                 220
   CTG GTG TTG GAC GAG GAC AAG AAG GAG GGT GGC AGT CAG TAC AGA TAT        822
   Leu Val Leu Asp Glu Asp Lys Lys Glu Gly Gly Ser Gln Tyr Arg Tyr
               225                 230                 235
   TTC ATT GTC GAA GAA GAA TGC CAC AAT GGA TAT GCG GCC TGT AAA AAC        870
   Phe Ile Val Glu Glu Glu Cys His Asn Gly Tyr Ala Ala Cys Lys Asn
           240                 245                 250
   AAG CTG AAA GAA GAT GCC TCG GTC AAA GAT GAG CGC AAA ACC GTC AGC        918
   Lys Leu Lys Glu Asp Ala Ser Val Lys Asp Glu Arg Lys Thr Val Ser
       255                 260                 265
   ACG CAG GAT TAT ACC GGC TCC AAC CGC TTA CTT GCG AAC CCG CTT GAG        966
   Thr Gln Asp Tyr Thr Gly Ser Asn Arg Leu Leu Ala Asn Pro Leu Glu
   270                 275                 280                 285
   TAT GGC AGC CAA TCA TGG CTG TTC CGA CCG GGT TGG CAT TTG GAC AAC       1014
   Tyr Gly Ser Gln Ser Trp Leu Phe Arg Pro Gly Trp His Leu Asp Asn
                   290                 295                 300
   CGC CAT TAT GTC GGA GCC GTT CTC GAA CGT ACG CAG CAG ACC TTT GAT       1062
   Arg His Tyr Val Gly Ala Val Leu Glu Arg Thr Gln Gln Thr Phe Asp
               305                 310                 315
   ACA CGG GAT ATG ACT GTT CCT GCC TAT TTT ACC AGT GAA GAT TAT GTA       1110
   Thr Arg Asp Met Thr Val Pro Ala Tyr Phe Thr Ser Glu Asp Tyr Val
           320                 325                 330
   CCC GGT TCG CTG AAA GGT CTT GGC AAA TAT TCG GGC GAT AAT AAG GCA     1158
   Pro Gly Ser Leu Lys Gly Leu Gly Lys Tyr Ser Gly Asp Asn Lys Ala
       335                 340                 345
   GAA AGG CTG TTT GTT CAG GGA GAG GGC AGT ACA TTG CAG GGT ATC GGT     1206
   Glu Arg Leu Phe Val Gln Gly Glu Gly Ser Thr Leu Gln Gly Ile Gly
   350                 355                 360                 365
   TAC GGT ACC GGC GTG TTT TAT GAT GAA CGC CAT ACT AAA AAC CGC TAC     1254
   Tyr Gly Thr Gly Val Phe Tyr Asp Glu Arg His Thr Lys Asn Arg Tyr
                   370                 375                 380
   GGG GTC GAA TAT GTT TAC CAT AAT GCT GAT AAG GAT ACC TGG GCC GAT     1302
   Gly Val Glu Tyr Val Tyr His Asn Ala Asp Lys Asp Thr Trp Ala Asp
               385                 390                 395
   TAC GCC CGA CTT TCT TAT GAC CGG CAA GGT ATA GAT TTG GAC AAC CGT     1350
   Tyr Ala Arg Leu Ser Tyr Asp Arg Gln Gly Ile Asp Leu Asp Asn Arg
           400                 405                 410
   TTG CAG CAG ACG CAT TGC TCT CAC GAC GGT TCG GAT AAA AAT TGC CGT     1398
   Leu Gln Gln Thr His Cys Ser His Asp Gly Ser Asp Lys Asn Cys Arg
       415                 420                 425
   CCC GAC GGC AAT AAA CCG TAT TCT TTC TAT AAA TCC GAC CGG ATG ATT     1446
   Pro Asp Gly Asn Lys Pro Tyr Ser Phe Tyr Lys Ser Asp Arg Met Ile
   430                 435                 440                 445
   TAT GAA GAA AGC CGA AAC CTG TTC CAA GCA GTA TTT AAA AAG GCA TTT     1494
   Tyr Glu Glu Ser Arg Asn Leu Phe Gln Ala Val Phe Lys Lys Ala Phe
                   450                 455                 460
   GAT ACG GCC AAA ATC CGT CAC AAT TTG AGT ATC AAT CTA GGG TAC GAC     1542
   Asp Thr Ala Lys Ile Arg His Asn Leu Ser Ile Asn Leu Gly Tyr Asp
               465                 470                 475
   CGC TTT AAG TCG CAA TTG TCC CAC AGC GAT TAT TAT CTT CAA AAC GCA     1590
   Arg Phe Lys Ser Gln Leu Ser His Ser Asp Tyr Tyr Leu Gln Asn Ala
           480                 485                 490
   GTT CAG GCA TAT GAT TTG ATA ACC CCG AAA AAG CCT CCG TTT CCC AAC     1638
   Val Gln Ala Tyr Asp Leu Ile Thr Pro Lys Lys Pro Pro Phe Pro Asn
       495                 500                 505
   GGA AGC AAA GAC AAC CCG TAT AGG GTG TCT ATC GGC AAG ACC ACG GTC     1686
   Gly Ser Lys Asp Asn Pro Tyr Arg Val Ser Ile Gly Lys Thr Thr Val
   510                 515                 520                 525
   AAT ACA TCG CCG ATA TGC CGT TTC GGC AAT AAC ACC TAT ACA GAC TGC     1734
   Asn Thr Ser Pro Ile Cys Arg Phe Gly Asn Asn Thr Tyr Thr Asp Cys
                   530                 535                 540
   ACA CCG AGG AAT ATC GGC GGC AAC GGT TAT TAT GCA GCC GTT CAA GAC    1782
   Thr Pro Arg Asn Ile Gly Gly Asn Gly Tyr Tyr Ala Ala Val Gln Asp
                   545                     550                     555
   AAT GTC CGT TTG GGC AGG TGG GCG GAT GTC GGA GCA GGC ATA CGT TAC    1830
   Asn Val Arg Leu Gly Arg Trp Ala Asp Val Gly Ala Gly Ile Arg Tyr
               560                     565                     570
   GAT TAC CGC AGC ACG CAT TCG GAA GAT AAG AGT GTC TCT ACC GGC ACT    1878
   Asp Tyr Arg Ser Thr His Ser Glu Asp Lys Ser Val Ser Thr Gly Thr
       575                     580                     585
   CAC CGC AAC CTT TCT TGG AAC GCG GGC GTA GTC CTC AAA CCT TTC ACC    1926
   His Arg Asn Leu Ser Trp Asn Ala Gly Val Val Leu Lys Pro Phe Thr
   590                     595                     600                     605
   TGG ATG GAT TTG ACT TAT CGC GCT TCT ACG GGC TTC CGT CTG CCG TCG    1974
   Trp Met Asp Leu Thr Tyr Arg Ala Ser Thr Gly Phe Arg Leu Pro Ser
                   610                     615                     620
   TTT GCC GAA ATG TAT GGC TGG AGA GCC GGG GAG TCT TTG AAA ACG TTG    2022
   Phe Ala Glu Met Tyr Gly Trp Arg Ala Gly Glu Ser Leu Lys Thr Leu
                   625                     630                     635
   GAT CTG AAA CCG GAA AAA TCC TTT AAT AGA GAG GCA GGT ATT GTA TTT    2070
   Asp Leu Lys Pro Glu Lys Ser Phe Asn Arg Glu Ala Gly Ile Val Phe
               640                     645                     650
   AAA GGG GAC TTC GGC AAT TTG GAA GCC AGC TAT TTC AAC AAT GCC TAT    2118
   Lys Gly Asp Phe Gly Asn Leu Glu Ala Ser Tyr Phe Asn Asn Ala Tyr
       655                     660                     665
   CGC GAC CTG ATT GCA TTC GGT TAT GAA ACC CGA ACT CAA AAC GGG CAA    2166
   Arg Asp Leu Ile Ala Phe Gly Tyr Glu Thr Arg Thr Gln Asn Gly Gln
   670                     675                     680                     685
   ACT TCG GCT TCT GGC GAC CCC GGA TAC CGA AAT GCC CAA AAT GCA CGG    2214
   Thr Ser Ala Ser Gly Asp Pro Gly Tyr Arg Asn Ala Gln Asn Ala Arg
                   690                     695                     700
   ATA GCC GGT ATC AAT ATT TTG GGT AAA ATC GAT TGG CAC GGC GTA TGG    2262
   Ile Ala Gly Ile Asn Ile Leu Gly Lys Ile Asp Trp His Gly Val Trp
                   705                     710                     715
   GGC GGG TTG CCG GAC GGG TTG TAT TCC ACG CTT GCC TAT AAC CGT ATC    2310
   Gly Gly Leu Pro Asp Gly Leu Tyr Ser Thr Leu Ala Tyr Asn Arg Ile
               720                     725                     730
   AAG GTC AAA GAT GCC GAT ATA CGC GCC GAC AGG ACG TTT GTA ACT TCA    2358
   Lys Val Lys Asp Ala Asp Ile Arg Ala Asp Arg Thr Phe Val Thr Ser
       735                     740                     745
   TAT CTC TTT GAT GCC GTC CAA CCT TCA CGA TAT GTA TTG GGT TTG GGT 2406
   Tyr Leu Phe Asp Ala Val Gln Pro Ser Arg Tyr Val Leu Gly Leu Gly
   750 755 760 765
   TAC GAC CAT CCT GAC GGA ATA TGG GGC ATC AAT ACG ATG TTT ACT TAT 2454
   Tyr Asp His Pro Asp Gly Ile Trp Gly Ile Asn Thr Met Phe Thr Tyr
   770 775 780
   TCC AAG GCA AAA TCT GTT GAC GAA CTG CTC GGC AGC CAG GCG CTG TTG 2502
   Ser Lys Ala Lys Ser Val Asp Glu Leu Leu Gly Ser Gln Ala Leu Leu
   785 790 795
   AAC GGT AAT GCC AAT GCT AAA AAA GCA GCA TCA CGG CGG ACG CGG CCT 2550
   Asn Gly Asn Ala Asn Ala Lys Lys Ala Ala Ser Arg Arg Thr Arg Pro
   800 805 810
   TGG TAT GTT ACG GAT GTT TCC GGA TAT TAC AAT ATC AAG AAA CAC CTG 2598
   Trp Tyr Val Thr Asp Val Ser Gly Tyr Tyr Asn Ile Lys Lys His Leu
   815 820 825
   ACC CTG CGC GCA GGT GTG TAC AAC CTC CTC AAC TAC CGC TAT GTT ACT 2646
   Thr Leu Arg Ala Gly Val Tyr Asn Leu Leu Asn Tyr Arg Tyr Val Thr
   830 835 840 845
   TGG GAA AAT GTG CGG CAA ACT GCC GGC GGC GCA GTC AAC CAA CAC AAA 2694
   Trp Glu Asn Val Arg Gln Thr Ala Gly Gly Ala Val Asn Gln His Lys
   850 855 860
   AAT GTC GGC GTT TAC AAC CGA TAT GCC GCC CCC GGC CGA AAC TAC ACA 2742
   Asn Val Gly Val Tyr Asn Arg Tyr Ala Ala Pro Gly Arg Asn Tyr Thr
   865 870 875
   TTT AGC TTG GAA ATG AAG TTT TAAACGTCCA AACGCCGCAA ATGCCGTCTG 2793
   Phe Ser Leu Glu Met Lys Phe
   880
   AAAGGCT 2800
(2) INFORMATION POUR LA SEQ ID NO: 4:
   (i) CARACTERISTIQUES DE LA SEQUENCE:
      (A) LONGUEUR: 908 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Met Gln Gln Gln His Leu Phe Arg Leu Asn Ile Leu Cys Leu Ser Leu
   -24 -20 -15 -10
   Met Thr Ala Leu Pro Val Tyr Ala Glu Asn Val Gln Ala Glu Gln Ala
                   -5                   1                   5
   Gln Glu Lys Gln Leu Asp Thr Ile Gln Val Lys Ala Lys Lys Gln Lys
        10                  15                  20
   Thr Arg Arg Asp Asn Glu Val Thr Gly Leu Gly Lys Leu Val Lys Ser
    25                  30                  35                  40
   Ser Asp Thr Leu Ser Lys Glu Gln Val Leu Asn Ile Arg Asp Leu Thr
                    45                  50                  55
   Arg Tyr Asp Pro Gly Ile Ala Val Val Glu Gln Gly Arg Gly Ala Ser
                60                  65                  70
   Ser Gly Tyr Ser Ile Arg Gly Met Asp Lys Asn Arg Val Ser Leu Thr
            75                  80                  85
   Val Asp Gly Val Ser Gln Ile Gln Ser Tyr Thr Ala Gln Ala Ala Leu
        90                  95                 100
   Gly Gly Thr Arg Thr Ala Gly Ser Ser Gly Ala Ile Asn Glu Ile Glu
   105                 110                 115                 120
   Tyr Glu Asn Val Lys Ala Val Glu Ile Ser Lys Gly Ser Asn Ser Ser
                   125                 130                 135
   Glu Tyr Gly Asn Gly Ala Leu Ala Gly Ser Val Ala Phe Gln Thr Lys
               140                 145                 150
   Thr Ala Ala Asp Ile Ile Gly Glu Gly Lys Gln Trp Gly Ile Gln Ser
           155                 160                 165
   Lys Thr Ala Tyr Ser Gly Lys Asp His Ala Leu Thr Gln Ser Leu Ala
       170                 175                 180
   Leu Ala Gly Arg Ser Gly Gly Ala Glu Ala Leu Leu Ile Tyr Thr Lys
   185                 190                 195                 200
   Arg Arg Gly Arg Glu Ile His Ala His Lys Asp Ala Gly Lys Gly Val
                   205                 210                 215
   Gln Ser Phe Asn Arg Leu Val Leu Asp Glu Asp Lys Lys Glu Gly Gly
               220                 225                 230
   Ser Gln Tyr Arg Tyr Phe Ile Val Glu Glu Glu Cys His Asn Gly Tyr
           235                 240                 245
   Ala Ala Cys Lys Asn Lys Leu Lys Glu Asp Ala Ser Val Lys Asp Glu
       250                 255                 260
   Arg Lys Thr Val Ser Thr Gln Asp Tyr Thr Gly Ser Asn Arg Leu Leu
   265 270 275 280
   Ala Asn Pro Leu Glu Tyr Gly Ser Gln Ser Trp Leu Phe Arg Pro Gly
   285 290 295
   Trp His Leu Asp Asn Arg His Tyr Val Gly Ala Val Leu Glu Arg Thr
   300 305 310
   Gln Gln Thr Phe Asp Thr Arg Asp Met Thr Val Pro Ala Tyr Phe Thr
   315 320 325
   Ser Glu Asp Tyr Val Pro Gly Ser Leu Lys Gly Leu Gly Lys Tyr Ser
   330 335 340
   Gly Asp Asn Lys Ala Glu Arg Leu Phe Val Gln Gly Glu Gly Ser Thr
   345 350 355 360
   Leu Gln Gly Ile Gly Tyr Gly Thr Gly Val Phe Tyr Asp Glu Arg His
   365 370 375
   Thr Lys Asn Arg Tyr Gly Val Glu Tyr Val Tyr His Asn Ala Asp Lys
   380 385 390
   Asp Thr Trp Ala Asp Tyr Ala Arg Leu Ser Tyr Asp Arg Gln Gly Ile
   395 400 405
   Asp Leu Asp Asn Arg Leu Gln Gln Thr His Cys Ser His Asp Gly Ser
   410 415 420
   Asp Lys Asn Cys Arg Pro Asp Gly Asn Lys Pro Tyr Ser Phe Tyr Lys
   425 430 435 440
   Ser Asp Arg Met Ile Tyr Glu Glu Ser Arg Asn Leu Phe Gln Ala Val
   445 450 455
   Phe Lys Lys Ala Phe Asp Thr Ala Lys Ile Arg His Asn Leu Ser Ile
   460 465 470
   Asn Leu Gly Tyr Asp Arg Phe Lys Ser Gln Leu Ser His Ser Asp Tyr
   475 480 485
   Tyr Leu Gln Asn Ala Val Gln Ala Tyr Asp Leu Ile Thr Pro Lys Lys
   490 495 500
   Pro Pro Phe Pro Asn Gly Ser Lys Asp Asn Pro Tyr Arg Val Ser Ile
   505 510 515 520
   Gly Lys Thr Thr Val Asn Thr Ser Pro Ile Cys Arg Phe Gly Asn Asn
   525 530 535
   Thr Tyr Thr Asp Cys Thr Pro Arg Asn Ile Gly Gly Asn Gly Tyr Tyr
   540 545 550
   Ala Ala Val Gln Asp Asn Val Arg Leu Gly Arg Trp Ala Asp Val Gly
   555 560 565
   Ala Gly Ile Arg Tyr Asp Tyr Arg Ser Thr His Ser Glu Asp Lys Ser
   570 575 580
   Val Ser Thr Gly Thr His Arg Asn Leu Ser Trp Asn Ala Gly Val Val
   585 590 595 600
   Leu Lys Pro Phe Thr Trp Met Asp Leu Thr Tyr Arg Ala Ser Thr Gly
   605 610 615
   Phe Arg Leu Pro Ser Phe Ala Glu Met Tyr Gly Trp Arg Ala Gly Glu
   620 625 630
   Ser Leu Lys Thr Leu Asp Leu Lys Pro Glu Lys Ser Phe Asn Arg Glu
   635 640 645
   Ala Gly Ile Val Phe Lys Gly Asp Phe Gly Asn Leu Glu Ala Ser Tyr
   650 655 660
   Phe Asn Asn Ala Tyr Arg Asp Leu Ile Ala Phe Gly Tyr Glu Thr Arg
   665 670 675 680
   Thr Gln Asn Gly Gln Thr Ser Ala Ser Gly Asp Pro Gly Tyr Arg Asn
   685 690 695
   Ala Gln Asn Ala Arg Ile Ala Gly Ile Asn Ile Leu Gly Lys Ile Asp
   700 705 710
   Trp His Gly Val Trp Gly Gly Leu Pro Asp Gly Leu Tyr Ser Thr Leu
   715 720 725
   Ala Tyr Asn Arg Ile Lys Val Lys Asp Ala Asp Ile Arg Ala Asp Arg
   730 735 740
   Thr Phe Val Thr Ser Tyr Leu Phe Asp Ala Val Gln Pro Ser Arg Tyr
   745 750 755 760
   Val Leu Gly Leu Gly Tyr Asp His Pro Asp Gly Ile Trp Gly Ile Asn
   765 770 775
   Thr Met Phe Thr Tyr Ser Lys Ala Lys Ser Val Asp Glu Leu Leu Gly
   780 785 790
   Ser Gln Ala Leu Leu Asn Gly Asn Ala Asn Ala Lys Lys Ala Ala Ser
   795 800 805
   Arg Arg Thr Arg Pro Trp Tyr Val Thr Asp Val Ser Gly Tyr Tyr Asn
   810 815 820
   Ile Lys Lys His Leu Thr Leu Arg Ala Gly Val Tyr Asn Leu Leu Asn
   825 830 835 840
   Tyr Arg Tyr Val Thr Trp Glu Asn Val Arg Gln Thr Ala Gly Gly Ala
   845 850 855
   Val Asn Gln His Lys Asn Val Gly Val Tyr Asn Arg Tyr Ala Ala Pro
   860 865 870
   Gly Arg Asn Tyr Thr Phe Ser Leu Glu Met Lys Phe
   875 880
(2) INFORMATION POUR LA SEQ ID NO: 5:
   (i) CARACTERISTIQUES DE LA SEQUENCE:
      (A) LONGUEUR: 2809 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: DNA codant pour la sous-unite Tbp1 du recepteur transferrin
      (B) SOUCHE: Neisseria meningitidis IM2169
   (ix) CARACTERISTIQUE ADDITIONELLE:
      (A) NOM/CLE: sig peptide
      (B) EMPLACEMENT: 71.. 142
   (ix) CARACTERISTIQUE ADDITIONELLE:
      (A) NOM/CLE: mat peptide
      (B) EMPLACEMENT: 143..2803
   (ix) CARACTERISTIQUE ADDITIONELLE:
      (A) NOM/CLE: CDS
      (B) EMPLACEMENT: 71..2803
   (xi) DESCRIPTION DE LA SEQUENCE: SEQ ID NO: 5:
   ATCAAGAATA AGGCTTCAGA CGGCATCGCT CCTTCCGATA CCGTCTGAAA GCGAAGATTA 60
   GGGAAACATT ATG CAA CAG CAA CAT TTG TTC CGA TTA AAT ATT TTA TGC 109
   Met Gln Gln Gln His Leu Phe Arg Leu Asn Ile Leu Cys
   -24 -20 -15
   CTG TCG CTG ATG ACT GCG CTG CCT GCT TAT GCA GAA AAT GTG CAA GCC 157
   Leu Ser Leu Met Thr Ala Leu Pro Ala Tyr Ala Glu Asn Val Gln Ala
   -10 -5 1 5
   GGA CAA GCA CAG GAA AAA CAG TTG GAT ACC ATA CAG GTA AAA GCC AAA 205
   Gly Gln Ala Gln Glu Lys Gln Leu Asp Thr Ile Gln Val Lys Ala Lys
   10 15 20
   AAA CAG AAA ACC CGC CGC GAT AAC GAA GTA ACC GGT CTG GGC AAA TTG 253
   Lys Gln Lys Thr Arg Arg Asp Asn Glu Val Thr Gly Leu Gly Lys Leu
   25 30 35
   GTC AAA ACC GCC GAC ACC CTC AGC AAG GAA CAG GTA CTC GAT ATC CGC 301
   Val Lys Thr Ala Asp Thr Leu Ser Lys Glu Gln Val Leu Asp Ile Arg
   40 45 50
   GAC CTG ACG CGT TAC GAC CCC GGC ATC GCC GTG GTC GAA CAG GGG CGC 349
   Asp Leu Thr Arg Tyr Asp Pro Gly Ile Ala Val Val Glu Gln Gly Arg
   55 60 65
   GGC GCA AGT TCG GGC TAC TCG ATA CGC GGT ATG GAC AAA AAC CGC GTT 397
   Gly Ala Ser Ser Gly Tyr Ser Ile Arg Gly Met Asp Lys Asn Arg Val
   70 75 80 85
   TCC TTG ACG GTG GAC GGC TTG GCG CAA ATA CAG TCC TAC ACC GCG CAG 445
   Ser Leu Thr Val Asp Gly Leu Ala Gln Ile Gln Ser Tyr Thr Ala Gln
   90 95 100
   GCG GCA TTG GGC GGG ACG AGG ACG GCG GGC AGC AGC GGC GCA ATC AAT 493
   Ala Ala Leu Gly Gly Thr Arg Thr Ala Gly Ser Ser Gly Ala Ile Asn
   105 110 115
   GAA ATC GAG TAT GAA AAC GTC AAA GCT GTC GAA ATC AGC AAA GGC TCA 541
   Glu Ile Glu Tyr Glu Asn Val Lys Ala Val Glu Ile Ser Lys Gly Ser
   120 125 130
   AAC TCG GTC GAA CAA GGC AGC GGC GCA TTG GCG GGT TCG GTC GCA TTT 589
   Asn Ser Val Glu Gln Gly Ser Gly Ala Leu Ala Gly Ser Val Ala Phe
   135 140 145
   CAA ACC AAA ACC GCC GAC GAT GTT ATC GGG GAA GGC AGG CAG TGG GGC 637
   Gln Thr Lys Thr Ala Asp Asp Val Ile Gly Glu Gly Arg Gln Trp Gly
   150 155 160 165
   ATT CAG AGT AAA ACC GCC TAT TCC GGC AAA AAC CGG GGG CTT ACC CAA 685
   Ile Gln Ser Lys Thr Ala Tyr Ser Gly Lys Asn Arg Gly Leu Thr Gln
   170 175 180
   TCC ATC GCG CTG GCG GGG CGC ATC GGC GGT GCG GAG GCT TTG CTG ATC 733
   Ser Ile Ala Leu Ala Gly Arg Ile Gly Gly Ala Glu Ala Leu Leu Ile
   185 190 195
   CAC ACC GGG CGG CGC GCG GGG GAA ATC CGC GCA CAC GAA GAT GCC GGA 781
   His Thr Gly Arg Arg Ala Gly Glu Ile Arg Ala His Glu Asp Ala Gly
   200 205 210
   CGC GGC GTT CAG AGC TTT AAC AGG CTG GTG CCG GTT GAA GAC AGC AGC 829
   Arg Gly Val Gln Ser Phe Asn Arg Leu Val Pro Val Glu Asp Ser Ser
   215 220 225
   GAA TAC GCC TAT TTC ATC GTT GAA GAT GAA TGC GAA GGC AAA AAT TAC 877
   Glu Tyr Ala Tyr Phe Ile Val Glu Asp Glu Cys Glu Gly Lys Asn Tyr
   230 235 240 245
   GAA ACG TGT AAA AGC AAA CCG AAA AAA GAT GTT GTC GGC AAA GAC GAA 925
   Glu Thr Cys Lys Ser Lys Pro Lys Lys Asp Val Val Gly Lys Asp Glu
   250 255 260
   CGT CAA ACG GTT TCC ACC CGA GAC TAC ACG GGC CCC AAC CGC TTC CTC 973
   Arg Gln Thr Val Ser Thr Arg Asp Tyr Thr Gly Pro Asn Arg Phe Leu
   265 270 275
   GCC GAT CCG CTT TCA TAC GAA AGC CGA TCG TGG CTG TTC CGC CCG GGT 1021
   Ala Asp Pro Leu Ser Tyr Glu Ser Arg Ser Trp Leu Phe Arg Pro Gly
   280 285 290
   TTT CGT TTT GAA AAC AAA CGG CAC TAC ATC GGC GGC ATA CTC GAA CAC 1069
   Phe Arg Phe Glu Asn Lys Arg His Tyr Ile Gly Gly Ile Leu Glu His
   295 300 305
   ACG CAA CAA ACT TTC GAC ACG CGC GAT ATG ACG GTT CCG GCA TTC CTG 1117
   Thr Gln Gln Thr Phe Asp Thr Arg Asp Met Thr Val Pro Ala Phe Leu
   310 315 320 325
   ACC AAG GCG GTT TTT GAT GCA AAT TCA AAA CAG GCG GGT TCT TTG CCC 1165
   Thr Lys Ala Val Phe Asp Ala Asn Ser Lys Gln Ala Gly Ser Leu Pro
   330 335 340
   GGC AAC GGC AAA TAC GCG GGC AAC CAC AAA TAC GGC GGA CTG TTT ACC 1213
   Gly Asn Gly Lys Tyr Ala Gly Asn His Lys Tyr Gly Gly Leu Phe Thr
   345 350 355
   AAC GGC GAA AAC GGT GCG CTG GTG GGC GCG GAA TAC GGT ACG GGC GTG 1261
   Asn Gly Glu Asn Gly Ala Leu Val Gly Ala Glu Tyr Gly Thr Gly Val
   360 365 370
   TTT TAC GAC GAG ACG CAC ACC AAA AGC CGC TAC GGT TTG GAA TAT GTC 1309
   Phe Tyr Asp Glu Thr His Thr Lys Ser Arg Tyr Gly Leu Glu Tyr Val
   375 380 385
   TAT ACC AAT GCC GAT AAA GAC ACT TGG GCG GAT TAT GCC CGC CTC TCT 1357
   Tyr Thr Asn Ala Asp Lys Asp Thr Trp Ala Asp Tyr Ala Arg Leu Ser
   390 395 400 405
   TAC GAC CGG CAG GGC ATC GGT TTG GAC AAT CAT TTT CAG CAG ACG CAC      1405
   Tyr Asp Arg Gln Gly Ile Gly Leu Asp Asn His Phe Gln Gln Thr His
                   410                     415                     420
   TGT TCT GCC GAC GGT TCG GAC AAA TAT TGC CGC CCG AGT GCC GAC AAG      1453
   Cys Ser Ala Asp Gly Ser Asp Lys Tyr Cys Arg Pro Ser Ala Asp Lys
               425                     430                     435
   CCG TTT TCC TAT TAC AAA TCC GAC CGC GTG ATT TAC GGG GAA AGC CAC      1501
   Pro Phe Ser Tyr Tyr Lys Ser Asp Arg Val Ile Tyr Gly Glu Ser His
           440                     445                     450
   AGG CTC TTG CAG GCG GCA TTC AAA AAA TCC TTC GAT ACC GCC AAA ATC      1549
   Arg Leu Leu Gln Ala Ala Phe Lys Lys Ser Phe Asp Thr Ala Lys Ile
       455                     460                     465
   CGC CAC AAC CTG AGC GTG AAT CTC GGG TTT GAC CGC TTT GAC TCT AAT      1597
   Arg His Asn Leu Ser Val Asn Leu Gly Phe Asp Arg Phe Asp Ser Asn
   470                     475                     480                     485
   CTC CGC CAT CAG GAT TAT TAT TAT CAA CAT GCC AAC CGC GCC TAT TCG      1645
   Leu Arg His Gln Asp Tyr Tyr Tyr Gln His Ala Asn Arg Ala Tyr Ser
                   490                     495                     500
   TCG AAA ACG CCC CCT AAA ACC GCC AAC CCC AAC GGC GAC AAG AGC AAA      1693
   Ser Lys Thr Pro Pro Lys Thr Ala Asn Pro Asn Gly Asp Lys Ser Lys
               505                     510                     515
   CCC TAT TGG GTC AGC ATA GGC GGG GGA AAT GTG GTT ACG GGG CAA ATC      1741
   Pro Tyr Trp Val Ser Ile Gly Gly Gly Asn Val Val Thr Gly Gln Ile
           520                     525                     530
   TGC CTC TTT GGC AAC AAT ACT TAT ACG GAC TGC ACG CCG CGC AGC ATC      1789
   Cys Leu Phe Gly Asn Asn Thr Tyr Thr Asp Cys Thr Pro Arg Ser Ile
       535                     540                     545
   AAC GGC AAA AGC TAT TAC GCG GCA GTT CGG GAC AAT GTC CGT TTG GGC      1837
   Asn Gly Lys Ser Tyr Tyr Ala Ala Val Arg Asp Asn Val Arg Leu Gly
   550                     555                     560                     565
   AGG TGG GCG GAT GTC GGC GCG GGG TTG CGC TAC GAC TAC CGC AGC ACG      1885
   Arg Trp Ala Asp Val Gly Ala Gly Leu Arg Tyr Asp Tyr Arg Ser Thr
                   570                     575                     580
   CAT TCG GAC GAC GGC AGC GTT TCC ACC GGC ACG CAC CGC ACC CTG TCC      1933
   His Ser Asp Asp Gly Ser Val Ser Thr Gly Thr His Arg Thr Leu Ser
               585                     590                     595
   TGG AAC GCC GGC ATC GTC CTC AAA CCT GCC GAC TGG CTG GAT TTG ACT      1981
   Trp Asn Ala Gly Ile Val Leu Lys Pro Ala Asp Trp Leu Asp Leu Thr
           600                     605                     610
   TAC CGC ACT TCA ACC GGC TTC CGC CTG CCC TCG TTT GCG GAA ATG TAC 2029
   Tyr Arg Thr Ser Thr Gly Phe Arg Leu Pro Ser Phe Ala Glu Met Tyr
   615 620 625
   GGC TGG CGG TCG GGT GTT CAA AGC AAG GCG GTC AAA ATC GAT CCG GAA 2077
   Gly Trp Arg Ser Gly Val Gln Ser Lys Ala Val Lys Ile Asp Pro Glu
   630 635 640 645
   AAA TCG TTC AAC AAA GAA GCC GGC ATC GTG TTT AAA GGC GAT TTC GGC 2125
   Lys Ser Phe Asn Lys Glu Ala Gly Ile Val Phe Lys Gly Asp Phe Gly
   650 655 660
   AAC TTG GAG GCA AGT TGG TTC AAC AAT GCC TAC CGC GAT TTG ATT GTC 2173
   Asn Leu Glu Ala Ser Trp Phe Asn Asn Ala Tyr Arg Asp Leu Ile Val
   665 670 675
   CGG GGT TAT GAA GCG CAA ATT AAA AAC GGC AAA GAA GAA GCC AAA GGC 2221
   Arg Gly Tyr Glu Ala Gln Ile Lys Asn Gly Lys Glu Glu Ala Lys Gly
   680 685 690
   GAC CCG GCT TAC CTC AAT GCC CAA AGC GCG CGG ATT ACC GGC ATC AAT 2269
   Asp Pro Ala Tyr Leu Asn Ala Gln Ser Ala Arg Ile Thr Gly Ile Asn
   695 700 705
   ATT TTG GGC AAA ATC GAT TGG AAC GGC GTA TGG GAT AAA TTG CCC GAA 2317
   Ile Leu Gly Lys Ile Asp Trp Asn Gly Val Trp Asp Lys Leu Pro Glu
   710 715 720 725
   GGT TGG TAT TCT ACA TTT GCC TAT AAT CGT GTC CAT GTC CGC GAC ATC 2365
   Gly Trp Tyr Ser Thr Phe Ala Tyr Asn Arg Val His Val Arg Asp Ile
   730 735 740
   AAA AAA CGC GCA GAC CGC ACC GAT ATT CAA TCA CAC CTG TTT GAT GCC 2413
   Lys Lys Arg Ala Asp Arg Thr Asp Ile Gln Ser His Leu Phe Asp Ala
   745 750 755
   ATC CAA CCC TCG CGC TAT GTC GTC GGC TTG GGC TAT GAC CAA CCG GAA 2461
   Ile Gln Pro Ser Arg Tyr Val Val Gly Leu Gly Tyr Asp Gln Pro Glu
   760 765 770
   GGC AAA TGG GGT GTG AAC GGT ATG CTG ACT TAT TCC AAA GCC AAG GAA 2509
   Gly Lys Trp Gly Val Asn Gly Met Leu Thr Tyr Ser Lys Ala Lys Glu
   775 780 785
   ATC ACA GAG TTG TTG GGC AGC CGG GCT TTG CTC AAC GGC AAC AGC CGC 2557
   Ile Thr Glu Leu Leu Gly Ser Arg Ala Leu Leu Asn Gly Asn Ser Arg
   790 795 800 805
   AAT ACA AAA GCC ACC GCG CGC CGT ACC CGC CCT TGG TAT ATT GTG GAT 2605
   Asn Thr Lys Ala Thr Ala Arg Arg Thr Arg Pro Trp Tyr Ile Val Asp
   810 815 820
   GTG TCC GGT TAT TAC ACG ATT AAA AAA CAC TTC ACC CTC CGT GCG GGC      2653
   Val Ser Gly Tyr Tyr Thr Ile Lys Lys His Phe Thr Leu Arg Ala Gly
               825                 830                 835
   GTG TAC AAC CTC CTC AAC TAC CGC TAT GTT ACT TGG GAA AAT GTG CGG      2701
   Val Tyr Asn Leu Leu Asn Tyr Arg Tyr Val Thr Trp Glu Asn Val Arg
           840                 845                 850
   CAA ACT GCC GGC GGC GCA GTC AAC CAA CAC AAA AAT GTC GGC GTT TAC      2749
   Gln Thr Ala Gly Gly Ala Val Asn Gln His Lys Asn Val Gly Val Tyr
       855                 860                 865
   AAC CGA TAT GCC GCC CCC GGC CGA AAC TAC ACA TTT AGC TTG GAA ATG      2797
   Asn Arg Tyr Ala Ala Pro Gly Arg Asn Tyr Thr Phe Ser Leu Glu Met
   870                 875                 880                 885
   AAG TTT TAAACG                                                       2809
   Lys Phe
(2) INFORMATION POUR LA SEQ ID NO: 6:
   (i) CARACTERISTIQUES DE LA SEQUENCE:
      (A) LONGUEUR: 911 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Gln Gln Gln His Leu Phe Arg Leu Asn Ile Leu Cys Leu Ser Leu
   -24             -20                 -15                 -10
   Met Thr Ala Leu Pro Ala Tyr Ala Glu Asn Val Gln Ala Gly Gln Ala
               -5                  1               5
   Gln Glu Lys Gln Leu Asp Thr Ile Gln Val Lys Ala Lys Lys Gln Lys
       10                  15                  20
   Thr Arg Arg Asp Asn Glu Val Thr Gly Leu Gly Lys Leu Val Lys Thr
   25                  30                  35                  40
   Ala Asp Thr Leu Ser Lys Glu Gln Val Leu Asp Ile Arg Asp Leu Thr
                   45                  50                  55
   Arg Tyr Asp Pro Gly Ile Ala Val Val Glu Gln Gly Arg Gly Ala Ser
               60                  65                  70
   Ser Gly Tyr Ser Ile Arg Gly Met Asp Lys Asn Arg Val Ser Leu Thr
           75                  80                  85
   Val Asp Gly Leu Ala Gln Ile Gln Ser Tyr Thr Ala Gln Ala Ala Leu
   90 95 100
   Gly Gly Thr Arg Thr Ala Gly Ser Ser Gly Ala Ile Asn Glu Ile Glu
   105 110 115 120
   Tyr Glu Asn Val Lys Ala Val Glu Ile Ser Lys Gly Ser Asn Ser Val
   125 130 135
   Glu Gln Gly Ser Gly Ala Leu Ala Gly Ser Val Ala Phe Gln Thr Lys
   140 145 150
   Thr Ala Asp Asp Val Ile Gly Glu Gly Arg Gln Trp Gly Ile Gln Ser
   155 160 165
   Lys Thr Ala Tyr Ser Gly Lys Asn Arg Gly Leu Thr Gln Ser Ile Ala
   170 175 180
   Leu Ala Gly Arg Ile Gly Gly Ala Glu Ala Leu Leu Ile His Thr Gly
   185 190 195 200
   Arg Arg Ala Gly Glu Ile Arg Ala His Glu Asp Ala Gly Arg Gly Val
   205 210 215
   Gln Ser Phe Asn Arg Leu Val Pro Val Glu Asp Ser Ser Glu Tyr Ala
   220 225 230
   Tyr Phe Ile Val Glu Asp Glu Cys Glu Gly Lys Asn Tyr Glu Thr Cys
   235 240 245
   Lys Ser Lys Pro Lys Lys Asp Val Val Gly Lys Asp Glu Arg Gln Thr
   250 255 260
   Val Ser Thr Arg Asp Tyr Thr Gly Pro Asn Arg Phe Leu Ala Asp Pro
   265 270 275 280
   Leu Ser Tyr Glu Ser Arg Ser Trp Leu Phe Arg Pro Gly Phe Arg Phe
   285 290 295
   Glu Asn Lys Arg His Tyr Ile Gly Gly Ile Leu Glu His Thr Gln Gln
   300 305 310
   Thr Phe Asp Thr Arg Asp Met Thr Val Pro Ala Phe Leu Thr Lys Ala
   315 320 325
   Val Phe Asp Ala Asn Ser Lys Gln Ala Gly Ser Leu Pro Gly Asn Gly
   330 335 340
   Lys Tyr Ala Gly Asn His Lys Tyr Gly Gly Leu Phe Thr Asn Gly Glu
   345 350 355 360
   Asn Gly Ala Leu Val Gly Ala Glu Tyr Gly Thr Gly Val Phe Tyr Asp
   365 370 375
   Glu Thr His Thr Lys Ser Arg Tyr Gly Leu Glu Tyr Val Tyr Thr Asn
   380 385 390
   Ala Asp Lys Asp Thr Trp Ala Asp Tyr Ala Arg Leu Ser Tyr Asp Arg
   395 400 405
   Gln Gly Ile Gly Leu Asp Asn His Phe Gln Gln Thr His Cys Ser Ala
   410 415 420
   Asp Gly Ser Asp Lys Tyr Cys Arg Pro Ser Ala Asp Lys Pro Phe Ser
   425 430 435 440
   Tyr Tyr Lys Ser Asp Arg Val Ile Tyr Gly Glu Ser His Arg Leu Leu
   445 450 455
   Gln Ala Ala Phe Lys Lys Ser Phe Asp Thr Ala Lys Ile Arg His Asn
   460 465 470
   Leu Ser Val Asn Leu Gly Phe Asp Arg Phe Asp Ser Asn Leu Arg His
   475 480 485
   Gln Asp Tyr Tyr Tyr Gln His Ala Asn Arg Ala Tyr Ser Ser Lys Thr
   490 495 500
   Pro Pro Lys Thr Ala Asn Pro Asn Gly Asp Lys Ser Lys Pro Tyr Trp
   505 510 515 520
   Val Ser Ile Gly Gly Gly Asn Val Val Thr Gly Gln Ile Cys Leu Phe
   525 530 535
   Gly Asn Asn Thr Tyr Thr Asp Cys Thr Pro Arg Ser Ile Asn Gly Lys
   540 545 550
   Ser Tyr Tyr Ala Ala Val Arg Asp Asn Val Arg Leu Gly Arg Trp Ala
   555 560 565
   Asp Val Gly Ala Gly Leu Arg Tyr Asp Tyr Arg Ser Thr His Ser Asp
   570 575 580
   Asp Gly Ser Val Ser Thr Gly Thr His Arg Thr Leu Ser Trp Asn Ala
   585 590 595 600
   Gly Ile Val Leu Lys Pro Ala Asp Trp Leu Asp Leu Thr Tyr Arg Thr
   605 610 615
   Ser Thr Gly Phe Arg Leu Pro Ser Phe Ala Glu Met Tyr Gly Trp Arg
   620 625 630
   Ser Gly Val Gln Ser Lys Ala Val Lys Ile Asp Pro Glu Lys Ser Phe
   635 640 645
   Asn Lys Glu Ala Gly Ile Val Phe Lys Gly Asp Phe Gly Asn Leu Glu
   650 655 660
   Ala Ser Trp Phe Asn Asn Ala Tyr Arg Asp Leu Ile Val Arg Gly Tyr
   665 670 675 680
   Glu Ala Gln Ile Lys Asn Gly Lys Glu Glu Ala Lys Gly Asp Pro Ala
   685 690 695
   Tyr Leu Asn Ala Gln Ser Ala Arg Ile Thr Gly Ile Asn Ile Leu Gly
   700 705 710
   Lys Ile Asp Trp Asn Gly Val Trp Asp Lys Leu Pro Glu Gly Trp Tyr
   715 720 725
   Ser Thr Phe Ala Tyr Asn Arg Val His Val Arg Asp Ile Lys Lys Arg
   730 735 740
   Ala Asp Arg Thr Asp Ile Gln Ser His Leu Phe Asp Ala Ile Gln Pro
   745 750 755 760
   Ser Arg Tyr Val Val Gly Leu Gly Tyr Asp Gln Pro Glu Gly Lys Trp
   765 770 775
   Gly Val Asn Gly Met Leu Thr Tyr Ser Lys Ala Lys Glu Ile Thr Glu
   780 785 790
   Leu Leu Gly Ser Arg Ala Leu Leu Asn Gly Asn Ser Arg Asn Thr Lys
   795 800 805
   Ala Thr Ala Arg Arg Thr Arg Pro Trp Tyr Ile Val Asp Val Ser Gly
   810 815 820
   Tyr Tyr Thr Ile Lys Lys His Phe Thr Leu Arg Ala Gly Val Tyr Asn
   825 830 835 840
   Leu Leu Asn Tyr Arg Tyr Val Thr Trp Glu Asn Val Arg Gln Thr Ala
   845 850 855
   Gly Gly Ala Val Asn Gln His Lys Asn Val Gly Val Tyr Asn Arg Tyr
   860 865 870
   Ala Ala Pro Gly Arg Asn Tyr Thr Phe Ser Leu Glu Met Lys Phe
   875 880 885
(2) INFORMATION POUR LA SEQ ID NO: 7:
   (i) CARACTERISTIQUES DE LA SEQUENCE:
      (A) LONGUEUR: 2230 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: DNA codant pour la sous-unité Tbp2 du recepteur transferrine
      (B) SOUCHE: Neisseria meningitidis IM2169
   (ix) CARACTERISTIQUE ADDITIONELLE:
      (A) NOM/CLE: sig peptide
      (B) EMPLACEMENT: 60..119
   (ix) CARACTERISTIQUE ADDITIONELLE:
      (A) NOM/CLE: mat peptide
      (B) EMPLACEMENT: 120..2192
   (ix) CARACTERISTIQUE ADDITIONELLE:
      (A) NOM/CLE: CDS
      (B) EMPLACEMENT: 60..2192
   (xi) DESCRIPTION DE LA SEQUENCE: SEQ ID NO: 7:
   ATTTGTTAAA AATAAATAAA ATAATAATCC TTATCATTCT TTAATTGAAT TGGGTTTAT        59
   ATG AAC AAT CCA TTG GTA AAT CAG GCT GCT ATG GTG CTG CCT GTG TTT        107
   Met Asn Asn Pro Leu Val Asn Gln Ala Ala Met Val Leu Pro Val Phe
   -20                 -15                 -10                  -5
   TTG TTG AGT GCC TGT CTG GGC GGC GGC GGC AGT TTC GAT CTT GAT TCT        155
   Leu Leu Ser Ala Cys Leu Gly Gly Gly Gly Ser Phe Asp Leu Asp Ser
                        1               5                  10
   GTC GAT ACC GAA GCC CCG CGT CCC GCG CCA AAG TAT CAA GAT GTT TCT        203
   Val Asp Thr Glu Ala Pro Arg Pro Ala Pro Lys Tyr Gln Asp Val Ser
            15                  20                  25
   TCC GAA AAA CCG CAA GCC CAA AAA GAC CAA GGC GGA TAC GGT TTT GCG        251
   Ser Glu Lys Pro Gln Ala Gln Lys Asp Gln Gly Gly Tyr Gly Phe Ala
        30                  35                  40
   ATG AGG TTG AAA CGG AGG AAT TGG TAT CCG GGG GCA GAA GAA AGC GAG        299
   Met Arg Leu Lys Arg Arg Asn Trp Tyr Pro Gly Ala Glu Glu Ser Glu
    45                  50                  55                  60
   GTT AAA CTG AAC GAG AGT GAT TGG GAG GCG ACG GGA TTG CCG ACA AAA        347
   Val Lys Leu Asn Glu Ser Asp Trp Glu Ala Thr Gly Leu Pro Thr Lys
                        65                  70                  75
   CCC AAG GAA CTT CCT AAA CGG CAA AAA TCG GTT ATT GAA AAA GTA GAA    395
   Pro Lys Glu Leu Pro Lys Arg Gln Lys Ser Val Ile Glu Lys Val Glu
               80                  85                  90
   ACA GAC GGC GAC AGC GAT ATT TAT TCT TCC CCC TAT CTC ACA CCA TCA    443
   Thr Asp Gly Asp Ser Asp Ile Tyr Ser Ser Pro Tyr Leu Thr Pro Ser
           95                  100                 105
   AAC CAT CAA AAC GGC AGC GCT GGC AAC GGT GTA AAT CAA CCT AAA AAT    491
   Asn His Gln Asn Gly Ser Ala Gly Asn Gly Val Asn Gln Pro Lys Asn
       110                 115                 120
   CAG GCA ACA GGT CAC GAA AAT TTC CAA TAT GTT TAT TCC GGT TGG TTT    539
   Gln Ala Thr Gly His Glu Asn Phe Gln Tyr Val Tyr Ser Gly Trp Phe
   125                 130                 135                 140
   TAT AAA CAT GCA GCG AGT GAA AAA GAT TTC AGT AAC AAA AAA ATT AAG    587
   Tyr Lys His Ala Ala Ser Glu Lys Asp Phe Ser Asn Lys Lys Ile Lys
                   145                 150                 155
   TCA GGC GAC GAT GGT TAT ATC TTC TAT CAC GGT GAA AAA CCT TCC CGA    635
   Ser Gly Asp Asp Gly Tyr Ile Phe Tyr His Gly Glu Lys Pro Ser Arg
               160                 165                 170
   CAA CTT CCT GCT TCT GGA AAA GTT ATC TAC AAA GGT GTG TGG CAT TTT    683
   Gln Leu Pro Ala Ser Gly Lys Val Ile Tyr Lys Gly Val Trp His Phe
           175                 180                 185
   GTA ACC GAT ACA AAA AAG GGT CAA GAT TTT CGT GAA ATT ATC CAG CCT    731
   Val Thr Asp Thr Lys Lys Gly Gln Asp Phe Arg Glu Ile Ile Gln Pro
       190                 195                 200
   TCA AAA AAA CAA GGC GAC AGG TAT AGC GGA TTT TCT GGT GAT GGC AGC    779
   Ser Lys Lys Gln Gly Asp Arg Tyr Ser Gly Phe Ser Gly Asp Gly Ser
   205                 210                 215                 220
   GAA GAA TAT TCC AAC AAA AAC GAA TCC ACG CTG AAA GAT GAT CAC GAG    827
   Glu Glu Tyr Ser Asn Lys Asn Glu Ser Thr Leu Lys Asp Asp His Glu
                   225                 230                 235
   GGT TAT GGT TTT ACC TCG AAT TTA GAA GTG GAT TTC GGC AAT AAG AAA    875
   Gly Tyr Gly Phe Thr Ser Asn Leu Glu Val Asp Phe Gly Asn Lys Lys
               240                 245                 250
   TTG ACG GGT AAA TTA ATA CGC AAT AAT GCG AGC CTA AAT AAT AAT ACT    923
   Leu Thr Gly Lys Leu Ile Arg Asn Asn Ala Ser Leu Asn Asn Asn Thr
           255                 260                 265
   AAT AAT GAC AAA CAT ACC ACC CAA TAC TAC AGC CTT GAT GCA CAA ATA    971
   Asn Asn Asp Lys His Thr Thr Gln Tyr Tyr Ser Leu Asp Ala Gln Ile
       270                 275                 280
   ACA GGC AAC CGC TTC AAC GGC ACG GCA ACG GCA ACT GAC AAA AAA GAG      1019
   Thr Gly Asn Arg Phe Asn Gly Thr Ala Thr Ala Thr Asp Lys Lys Glu
   285                 290                 295                 300
   AAT GAA ACC AAA CTA CAT CCC TTT GTT TCC GAC TCG TCT TCT TTG AGC      1067
   Asn Glu Thr Lys Leu His Pro Phe Val Ser Asp Ser Ser Ser Leu Ser
                   305                 310                 315
   GGC GGC TTT TTC GGC CCG CAG GGT GAG GAA TTG GGT TTC CGC TTT TTG      1115
   Gly Gly Phe Phe Gly Pro Gln Gly Glu Glu Leu Gly Phe Arg Phe Leu
               320                 325                 330
   AGC GAC GAT CAA AAA GTT GCC GTT GTC GGC AGC GCG AAA ACC AAA GAC      1163
   Ser Asp Asp Gln Lys Val Ala Val Val Gly Ser Ala Lys Thr Lys Asp
           335                 340                 345
   AAA CTG GAA AAT GGC GCG GCG GCT TCA GGC AGC ACA GGT GCG GCA GCA      1211
   Lys Leu Glu Asn Gly Ala Ala Ala Ser Gly Ser Thr Gly Ala Ala Ala
       350                 355                 360
   TCG GGC GGT GCG GCA GGC ACG TCG TCT GAA AAC AGT AAG CTG ACC ACG      1259
   Ser Gly Gly Ala Ala Gly Thr Ser Ser Glu Asn Ser Lys Leu Thr Thr
   365                 370                 375                 380
   GTT TTG GAT GCG GTT GAA TTG ACA CTA AAC GAC AAG AAA ATC AAA AAT      1307
   Val Leu Asp Ala Val Glu Leu Thr Leu Asn Asp Lys Lys Ile Lys Asn
                   385                 390                 395
   CTC GAC AAC TTC AGC AAT GCC GCC CAA CTG GTT GTC GAC GGC ATT ATG      1355
   Leu Asp Asn Phe Ser Asn Ala Ala Gln Leu Val Val Asp Gly Ile Met
               400                 405                 410
   ATT CCG CTC CTG CCC AAG GAT TCC GAA AGC GGG AAC ACT CAG GCA GAT      1403
   Ile Pro Leu Leu Pro Lys Asp Ser Glu Ser Gly Asn Thr Gln Ala Asp
           415                 420                 425
   AAA GGT AAA AAC GGC GGA ACA GAA TTT ACC CGC AAA TTT GAA CAC ACG      1451
   Lys Gly Lys Asn Gly Gly Thr Glu Phe Thr Arg Lys Phe Glu His Thr
       430                 435                 440
   CCG GAA AGT GAT AAA AAA GAC GCC CAA GCA GGT ACG CAG ACG AAT GGG      1499
   Pro Glu Ser Asp Lys Lys Asp Ala Gln Ala Gly Thr Gln Thr Asn Gly
   445                 450                 455                 460
   GCG CAA ACC GCT TCA AAT ACG GCA GGT GAT ACC AAT GGC AAA ACA AAA      1547
   Ala Gln Thr Ala Ser Asn Thr Ala Gly Asp Thr Asn Gly Lys Thr Lys
                   465                 470                 475
   ACC TAT GAA GTC GAA GTC TGC TGT TCC AAC CTC AAT TAT CTG AAA TAC      1595
   Thr Tyr Glu Val Glu Val Cys Cys Ser Asn Leu Asn Tyr Leu Lys Tyr
               480                 485                 490
   GGA ATG TTG ACG CGC AAA AAC AGC AAG TCC GCG ATG CAG GCA GGA GGA        1643
   Gly Met Leu Thr Arg Lys Asn Ser Lys Ser Ala Met Gln Ala Gly Gly
           495                 500                 505
   AAC AGT AGT CAA GCT GAT GCT AAA ACG GAA CAA GTT GAA CAA AGT ATG        1691
   Asn Ser Ser Gln Ala Asp Ala Lys Thr Glu Gln Val Glu Gln Ser Met
       510                 515                 520
   TTC CTC CAA GGC GAG CGT ACC GAT GAA AAA GAG ATT CCA ACC GAC CAA        1739
   Phe Leu Gln Gly Glu Arg Thr Asp Glu Lys Glu Ile Pro Thr Asp Gln
   525                 530                 535                 540
   AAC GTC GTT TAT CGG GGG TCT TGG TAC GGG CAT ATT GCC AAC GGC ACA        1787
   Asn Val Val Tyr Arg Gly Ser Trp Tyr Gly His Ile Ala Asn Gly Thr
                   545                 550                 555
   AGC TGG AGC GGC AAT GCT TCT GAT AAA GAG GGC GGC AAC AGG GCG GAA        1835
   Ser Trp Ser Gly Asn Ala Ser Asp Lys Glu Gly Gly Asn Arg Ala Glu
               560                 565                 570
   TTT ACT GTG AAT TTT GCC GAT AAA AAA ATT ACC GGC AAG TTA ACC GCT        1883
   Phe Thr Val Asn Phe Ala Asp Lys Lys Ile Thr Gly Lys Leu Thr Ala
           575                 580                 585
   GAA AAC AGG CAG GCG CAA ACC TTT ACC ATT GAG GGA ATG ATT CAG GGC        1931
   Glu Asn Arg Gln Ala Gln Thr Phe Thr Ile Glu Gly Met Ile Gln Gly
       590                 595                 600
   AAC GGC TTT GAA GGT ACG GCG AAA ACT GCT GAG TCA GGT TTT GAT CTC        1979
   Asn Gly Phe Glu Gly Thr Ala Lys Thr Ala Glu Ser Gly Phe Asp Leu
   605                 610                 615                 620
   GAT CAA AAA AAT ACC ACC CGC ACG CCT AAG GCA TAT ATC ACA GAT GCC        2027
   Asp Gln Lys Asn Thr Thr Arg Thr Pro Lys Ala Tyr Ile Thr Asp Ala
                   625                 630                 635
   AAG GTA AAG GGC GGT TTT TAC GGG CCT AAA GCC GAA GAG TTG GGC GGA        2075
   Lys Val Lys Gly Gly Phe Tyr Gly Pro Lys Ala Glu Glu Leu Gly Gly
               640                 645                 650
   TGG TTT GCC TAT CCG GGC GAT AAA CAA ACG GAA AAG GCA ACA GCT ACA        2123
   Trp Phe Ala Tyr Pro Gly Asp Lys Gln Thr Glu Lys Ala Thr Ala Thr
           655                 660                 665
   TCC AGC GAT GGA AAT TCA GCA AGC AGC GCG ACC GTG GTA TTC GGT GCG        2171
   Ser Ser Asp Gly Asn Ser Ala Ser Ser Ala Thr Val Val Phe Gly Ala
       670                 675                 680
   AAA CGC CAA CAG CCT GTG CAA TAAGCACGGT TGCCGAACAA TCAAGAATAA           2222
   Lys Arg Gln Gln Pro Val Gln
   685                 690
   GGCTTCAG 2230
(2) INFORMATION POUR LA SEQ ID NO: 8:
   (i) CARACTERISTIQUES DE LA SEQUENCE:
      (A) LONGUEUR: 711 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Asn Asn Pro Leu Val Asn Gln Ala Ala Met Val Leu Pro Val Phe
   -20 -15 -10 -5
   Leu Leu Ser Ala Cys Leu Gly Gly Gly Gly Ser Phe Asp Leu Asp Ser
   1 5 10
   Val Asp Thr Glu Ala Pro Arg Pro Ala Pro Lys Tyr Gln Asp Val Ser
   15 20 25
   Ser Glu Lys Pro Gln Ala Gln Lys Asp Gln Gly Gly Tyr Gly Phe Ala
   30 35 40
   Met Arg Leu Lys Arg Arg Asn Trp Tyr Pro Gly Ala Glu Glu Ser Glu
   45 50 55 60
   Val Lys Leu Asn Glu Ser Asp Trp Glu Ala Thr Gly Leu Pro Thr Lys
   65 70 75
   Pro Lys Glu Leu Pro Lys Arg Gln Lys Ser Val Ile Glu Lys Val Glu
   80 85 90
   Thr Asp Gly Asp Ser Asp Ile Tyr Ser Ser Pro Tyr Leu Thr Pro Ser
   95 100 105
   Asn His Gln Asn Gly Ser Ala Gly Asn Gly Val Asn Gln Pro Lys Asn
   110 115 120
   Gln Ala Thr Gly His Glu Asn Phe Gln Tyr Val Tyr Ser Gly Trp Phe
   125 130 135 140
   Tyr Lys His Ala Ala Ser Glu Lys Asp Phe Ser Asn Lys Lys Ile Lys
   145 150 155
   Ser Gly Asp Asp Gly Tyr Ile Phe Tyr His Gly Glu Lys Pro Ser Arg
   160 165 170
   Gln Leu Pro Ala Ser Gly Lys Val Ile Tyr Lys Gly Val Trp His Phe
   175 180 185
   Val Thr Asp Thr Lys Lys Gly Gln Asp Phe Arg Glu Ile Ile Gln Pro
   190 195 200
   Ser Lys Lys Gln Gly Asp Arg Tyr Ser Gly Phe Ser Gly Asp Gly Ser
   205 210 215 220
   Glu Glu Tyr Ser Asn Lys Asn Glu Ser Thr Leu Lys Asp Asp His Glu
   225 230 235
   Gly Tyr Gly Phe Thr Ser Asn Leu Glu Val Asp Phe Gly Asn Lys Lys
   240 245 250
   Leu Thr Gly Lys Leu Ile Arg Asn Asn Ala Ser Leu Asn Asn Asn Thr
   255 260 265
   Asn Asn Asp Lys His Thr Thr Gln Tyr Tyr Ser Leu Asp Ala Gln Ile
   270 275 280
   Thr Gly Asn Arg Phe Asn Gly Thr Ala Thr Ala Thr Asp Lys Lys Glu
   285 290 295 300
   Asn Glu Thr Lys Leu His Pro Phe Val Ser Asp Ser Ser Ser Leu Ser
   305 310 315
   Gly Gly Phe Phe Gly Pro Gln Gly Glu Glu Leu Gly Phe Arg Phe Leu
   320 325 330
   Ser Asp Asp Gln Lys Val Ala Val Val Gly Ser Ala Lys Thr Lys Asp
   335 340 345
   Lys Leu Glu Asn Gly Ala Ala Ala Ser Gly Ser Thr Gly Ala Ala Ala
   350 355 360
   Ser Gly Gly Ala Ala Gly Thr Ser Ser Glu Asn Ser Lys Leu Thr Thr
   365 370 375 380
   Val Leu Asp Ala Val Glu Leu Thr Leu Asn Asp Lys Lys Ile Lys Asn
   385 390 395
   Leu Asp Asn Phe Ser Asn Ala Ala Gln Leu Val Val Asp Gly Ile Met
   400 405 410
   Ile Pro Leu Leu Pro Lys Asp Ser Glu Ser Gly Asn Thr Gln Ala Asp
   415 420 425
   Lys Gly Lys Asn Gly Gly Thr Glu Phe Thr Arg Lys Phe Glu His Thr
   430 435 440
   Pro Glu Ser Asp Lys Lys Asp Ala Gln Ala Gly Thr Gln Thr Asn Gly
   445 450 455 460
   Ala Gln Thr Ala Ser Asn Thr Ala Gly Asp Thr Asn Gly Lys Thr Lys
                   465                 470                 475
   Thr Tyr Glu Val Glu Val Cys Cys Ser Asn Leu Asn Tyr Leu Lys Tyr
               480                 485                 490
   Gly Met Leu Thr Arg Lys Asn Ser Lys Ser Ala Met Gln Ala Gly Gly
           495                 500                 505
   Asn Ser Ser Gln Ala Asp Ala Lys Thr Glu Gln Val Glu Gln Ser Met
       510                 515                 520
   Phe Leu Gln Gly Glu Arg Thr Asp Glu Lys Glu Ile Pro Thr Asp Gln
   525                 530                 535                 540
   Asn Val Val Tyr Arg Gly Ser Trp Tyr Gly His Ile Ala Asn Gly Thr
                   545                 550                 555
   Ser Trp Ser Gly Asn Ala Ser Asp Lys Glu Gly Gly Asn Arg Ala Glu
               560                 565                 570
   Phe Thr Val Asn Phe Ala Asp Lys Lys Ile Thr Gly Lys Leu Thr Ala
           575                 580                 585
   Glu Asn Arg Gln Ala Gln Thr Phe Thr Ile Glu Gly Met Ile Gln Gly
       590                 595                 600
   Asn Gly Phe Glu Gly Thr Ala Lys Thr Ala Glu Ser Gly Phe Asp Leu
   605                 610                 615                 620
   Asp Gln Lys Asn Thr Thr Arg Thr Pro Lys Ala Tyr Ile Thr Asp Ala
                   625                 630                 635
   Lys Val Lys Gly Gly Phe Tyr Gly Pro Lys Ala Glu Glu Leu Gly Gly
               640                 645                 650
   Trp Phe Ala Tyr Pro Gly Asp Lys Gln Thr Glu Lys Ala Thr Ala Thr
           655                 660                 665
   Ser Ser Asp Gly Asn Ser Ala Ser Ser Ala Thr Val Val Phe Gly Ala
       670                 675                 680
   Lys Arg Gln Gln Pro Val Gln
   685                 690
(2) INFORMATION POUR LA SEQ ID NO: 9:
   (i) CARACTERISTIQUES DE LA SEQUENCE:
      (A) LONGUEUR: 51 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..51
   (ix) CARACTERISTIQUE ADDITIONELLE:
      (A) NOM/CLE: sig peptide
      (B) EMPLACEMENT: 1..51
   (xi) DESCRIPTION DE LA SEQUENCE: SEQ ID NO: 9:
   ATG AGG AAA AGA TTT TTT GTG GGA ATA TTC GCG ATA AAC CTC CTT GTT      48
   Met Arg Lys Arg Phe Phe Val Gly Ile Phe Ala Ile Asn Leu Leu Val
     1               5                  10                  15
   GGA                                                                  51
   Gly
(2) INFORMATION POUR LA SEQ ID NO: 10:
   (i) CARACTERISTIQUES DE LA SEQUENCE:
      (A) LONGUEUR: 17 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10:
   Met Arg Lys Arg Phe Phe Val Gly Ile Phe Ala Ile Asn Leu Leu Val
     1               5                  10                  15
   Gly
(2) INFORMATION POUR LA SEQ ID NO: 11:
   (i) CARACTERISTIQUES DE LA SEQUENCE:
      (A) LONGUEUR: 57 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..57
   (ix) CARACTERISTIQUE ADDITIONELLE:
      (A) NOM/CLE: sig peptide
      (B) EMPLACEMENT: 1..57
   (xi) DESCRIPTION DE LA SEQUENCE: SEQ ID NO: 11:
   ATG AAA AAA ATA ACA GGG ATT ATT TTA TTG CTT CTT GCA GTC ATT ATT      48
   Met Lys Lys Ile Thr Gly Ile Ile Leu Leu Leu Leu Ala Val Ile Ile
    1               5                  10                  15
   CTG TCT GCA                                                          57
   Leu Ser Ala
(2) INFORMATION POUR LA SEQ ID NO: 12:
   (i) CARACTERISTIQUES DE LA SEQUENCE:
      (A) LONGUEUR: 19 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 12:
   Met Lys Lys Ile Thr Gly Ile Ile Leu Leu Leu Leu Ala Val Ile Ile
    1               5                  10                  15
   Leu Ser Ala
(2) INFORMATION POUR LA SEQ ID NO: 13:
   (i) CARACTERISTIQUES DE LA SEQUENCE:
      (A) LONGUEUR: 60 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..60
   (ix) CARACTERISTIQUE ADDITIONELLE:
      (A) NOM/CLE: sig peptide
      (B) EMPLACEMENT: 1..60
   (xi) DESCRIPTION DE LA SEQUENCE: SEQ ID NO: 13:
   ATG AAA GCT ACT AAA CTG GTA CTG GGC GCG GTA ATC CTG GGT TCT ACT        48
   Met Lys Ala Thr Lys Leu Val Leu Gly Ala Val Ile Leu Gly Ser Thr
     1               5                  10                  15
   CTG CTG GCA GGT                                                        60
   Leu Leu Ala Gly
                20
(2) INFORMATION POUR LA SEQ ID NO: 14:
   (i) CARACTERISTIQUES DE LA SEQUENCE:
      (A) LONGUEUR: 20 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 14:
   Met Lys Ala Thr Lys Leu Val Leu Gly Ala Val Ile Leu Gly Ser Thr
     1               5                  10                  15
   Leu Leu Ala Gly
                20
(2) INFORMATION POUR LA SEQ ID NO: 15:
   (i) CARACTERISTIQUES DE LA SEQUENCE:
      (A) LONGUEUR: 69 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..69
   (ix) CARACTERISTIQUE ADDITIONELLE:
      (A) NOM/CLE: sig peptide
      (B) EMPLACEMENT: 1..69
   (xi) DESCRIPTION DE LA SEQUENCE: SEQ ID NO: 15:
   ATG AAA CTG ACA ACA CAT CAT CTA CGG ACA GGG GCC GCA TTA TTG GTG     48
   Met Lys Leu Thr Thr His His Leu Arg Thr Gly Ala Ala Leu Leu Val
     1               5                  10                  15
   GCC GGA ATT CTG CTG GCA GGT                                         69
   Ala Gly Ile Leu Leu Ala Gly
                20
(2) INFORMATION POUR LA SEQ ID NO: 16:
   (i) CARACTERISTIQUES DE LA SEQUENCE:
      (A) LONGUEUR: 23 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 16:
   Met Lys Leu Thr Thr His His Leu Arg Thr Gly Ala Ala Leu Leu Val
     1               5                  10                  15
   Ala Gly Ile Leu Leu Ala Gly
                20
(2) INFORMATION POUR LA SEQ ID NO: 17:
   (i) CARACTERISTIQUES DE LA SEQUENCE:
      (A) LONGUEUR: 69 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..69
   (ix) CARACTERISTIQUE ADDITIONELLE:
      (A) NOM/CLE: sig peptide
      (B) EMPLACEMENT: 1..69
   (xi) DESCRIPTION DE LA SEQUENCE: SEQ ID NO: 17:
   ATG TTT GTA ACG AGC AAA AAA ATG ACC GCG GCT GTT CTG GCA ATT ACT      48
   Met Phe Val Thr Ser Lys Lys Met Thr Ala Ala Val Leu Ala Ile Thr
     1             5                  10                  15
   TTG GCA ATG TCT CTG AGT GCA                                         69
   Leu Ala Met Ser Leu Ser Ala
               20
(2) INFORMATION POUR LA SEQ ID NO: 18:
   (i) CARACTERISTIQUES DE LA SEQUENCE:
      (A) LONGUEUR: 23 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 18:
   Met Phe Val Thr Ser Lys Lys Met Thr Ala Ala Val Leu Ala Ile Thr
     1             5                  10                  15
   Leu Ala Met Ser Leu Ser Ala
               20
(2) INFORMATION POUR LA SEQ ID NO: 19:
   (i) CARACTERISTIQUES DE LA SEQUENCE:
      (A) LONGUEUR: 63 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..63
   (ix) CARACTERISTIQUE ADDITIONELLE:
      (A) NOM/CLE: sig peptide
      (B) EMPLACEMENT: 1..63
   (xi) DESCRIPTION DE LA SEQUENCE: SEQ ID NO: 19:
   ATG CAA CTG AAC AAA GTG CTG AAA GGG CTG ATG ATT GCT CTG CCT GTT          48
   Met Gln Leu Asn Lys Val Leu Lys Gly Leu Met Ile Ala Leu Pro Val
     1               5                  10                  15
   ATG GCA ATT GCG GCA                                                      63
   Met Ala Ile Ala Ala
                    20
(2) INFORMATION POUR LA SEQ ID NO: 20:
   (i) CARACTERISTIQUES DE LA SEQUENCE:
      (A) LONGUEUR: 21 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 20:
   Met Gln Leu Asn Lys Val Leu Lys Gly Leu Met Ile Ala Leu Pro Val
     1               5                  10                  15
   Met Ala Ile Ala Ala
                    20
(2) INFORMATION POUR LA SEQ ID NO: 21:
   (i) CARACTERISTIQUES DE LA SEQUENCE:
      (A) LONGUEUR: 54 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) MOM/CLE: CDS
      (B) EMPLACEMENT: 1..54
   (ix) CARACTERISTIQUE ADDITIONELLE:
      (A) NOM/CLE: sig peptide
      (B) EMPLACEMENT: 1..54
   (xi) DESCRIPTION DE LA SEQUENCE: SEQ ID NO: 21:
   ATG AGA TAC CTG GCA ACA TTG TTG TTA TCT CTG GCG GTG TTA ATC ACC        48
   Met Arg Tyr Leu Ala Thr Leu Leu Leu Ser Leu Ala Val Leu Ile Thr
     1               5                  10                  15
   GCC GGG                                                                54
   Ala Gly
(2) INFORMATION POUR LA SEQ ID NO: 22:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 22:
   Met Arg Tyr Leu Ala Thr Leu Leu Leu Ser Leu Ala Val Leu Ile Thr
     1               5                  10                  15
   Ala Gly
(2) INFORMATION POUR LA SEQ ID NO: 23:
   (i) CARACTERISTIQUES DE LA SEQUENCE:
      (A) LONGUEUR: 66 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..66
   (ix) CARACTERISTIQUE ADDITIONELLE:
      (A) NOM/CLE: sig peptide
      (B) EMPLACEMENT: 1..66
   (xi) DESCRIPTION DE LA SEQUENCE: SEQ ID NO: 23:
   ATG AAA CAT AAC GTT AAG CTG ATG GCA ATG ACT GCC GTT TTA TCC TCT      48
   Met Lys His Asn Val Lys Leu Met Ala Met Thr Ala Val Leu Ser Ser
     1               5                  10                  15
   GTC CTC GTG CTC TCC GGG                                              66
   Val Leu Val Leu Ser Gly
               20
(2) INFORMATION POUR LA SEQ ID NO: 24:
   (i) CARACTERISTIQUES DE LA SEQUENCE:
      (A) LONGUEUR: 22 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 24:
   Met Lys His Asn Val Lys Leu Met Ala Met Thr Ala Val Leu Ser Ser
     1               5                  10                  15
   Val Leu Val Leu Ser Gly
               20